# EUROPEAN PATENT APPLICATION

(11) **EP 3 449 814 A1**
(43) Date of publication of application: **06.03.2019**
(21) Application number: 17778694.4
(22) Date of filing: 07.04.2017
(51) Int. Cl.: A61B 5/00

(54) **SENSING SYSTEM UTILIZING MULTIFUNCTIONAL FABRIC, METHOD, AND OBJECT**

(30) Priority: 07.04.2016 US 201662319304 P
(71) Applicant: Yang, Chang Ming, Miaoli 350 (TW)
(72) Inventor: Yang, Chang Ming, Miaoli 350 (TW)
(74) Representative: Osha Liang
(86) International application number: PCT/CN2017/079741
(87) International publication number: WO 2017/174031

(57) **Abstract**

A sensing system utilizing a multifunctional fabric provides capabilities of self-monitoring and monitoring the ambient environment before using the sensing fabric, and consequently, the system can determine whether the fabric is operating properly before using the fabric. The system can continuously provide information for determining a previous sensing result as well as continuously monitoring a signal of interest. If a problem occurs in any interval, the system can notify a user or a monitoring center of the problem, and resolves the problem so as to provide an uninterrupted monitored signal, achieving full-time monitoring. As a result, it is not required to frequently replace the sensing fabric, resulting in an environmental friendly effect. More importantly, the system has an energy generation mechanism and can conserve the energy. The system is applicable to a human being or animal. The monitored items include: a heartbeat, breathing, temperature, perspiration, blood pressure, blood oxygen, posture, gait, excretion, bleeding detection, and others. A pattern or body paint can be applied to the skin to serve as a transmission line or electrode, or provide an insulation effect, or provide an effect of discharging static electricity. The system on the fabric can interact with it. In other words, the two can operate in parallel. This also serves as a backup. Moreover, static electricity on bodies, fabrics, shoes, and chairs can be monitored, and the static electricity can be removed even stored. An electrostatic effect can be utilized to attract the fabric to a body and measure a physiological signal. Also, electricity can be generated by magnetic induction. The system is applicable in entertainment and leisure, and can be used to identify human being or animals.

## Description

### FIELD OF THE INVENTION

The present invention relates to a system, method, and article utilizing an intelligent sensing fabric, which can be used for detecting physiological function, posture, state of the ambient environment and state of the sensing fabric itself, and can be used in sports medicine, fitness, health care, entertainment, industrial safety and other fields. The system starts to sense itself and the surrounding environment since leaving the factory, and the information in use can be accumulated and recorded continuously. Finally, if there is a problem with the system, the user or the monitoring center can be informed. Thus, the function of an intelligent fabric sensing system is achieved.

### BACKGROUND ART

As disclosed in US Patent No. 201500126834 A1, patterns have been used on fabrics or skin as a sensor. We use a pattern or body painting applied to the skin as a transmission wire or an electrode, which can produce insulation effect or the effect of generating and eliminating static electricity. The system on the fabric can interact with it. In other words, the two can operate in parallel. It is also a back-up.

US Patent No. 8193465 B2 discloses a fabric having a switch thereon and a sensor on the switch; or the switch is used on fabrics to detect changes in posture.

US Patent No. 20100170704 A1 discloses a fabric having a crevice. The crevice has conductive materials on its two sides, which can change and detect its change with an external force.

US Patent No. 8331097 B2 discloses a fabric that can be used as a capacitor; the capacitor is chargeable and a battery can be used. Accessories are not necessarily disposed on the same piece of fabric, but can be separately disposed on two pieces of fabrics. Meanwhile, electromagnetic induction can be realized, and there is also a reference area to detect leakage. Said patent does not mention that electromagnetic induction can be achieved on a single piece of fabric.

US Patent No. 7750790 B2 relates to a strain gauge. When an external force acts on the fabric, the geometrical properties of the at least one conductive yarn alter so that a corresponding change of an electric property is sensed. Said sensing is impedance (resistance, capacitance, inductance), or magnetic flux or electric flux is sensed. In our present patent, it can be a transmission wire, it can be converted to energy for storage, and it can also be used as an antenna for wireless transmission.

US Patent No. 20130066168 A1 discloses a system for generating physiological signals using a fabric capacitive sensor. A capacitive sensor is formed between a conductive fabric and a human body. No details are given as to the effect of static electricity between the human body and the fabric.

US Patent No. 20140343392 A1 discloses heartbeat detection or whether electrodes are in good contact. Dry electrode or capacitive coupling is based on a change in state between the electrode and the environment or the human body to choose which signal is used for processing. The electrode or the fabric, connecting wire, suspension strip around the electrode can be used as an antenna. To measure the impedance between the electrode and the body surface, the impedance between the two electrodes and body surface rather than the impedance between a single electrode and the body surface is measured.

US Patent No. 2012015076 A1 discloses a technique for detecting physiological function and posture. According to said patent, only one physiological signal is read at a time.

US Patent No. 20140084045 A1 discloses pattern-making, packaging and manufacturing of fabric electronization.

US Patent No. US 20110282164 A1 discloses a sensing device comprising a substrate material layer and a plurality of sensors provided on the substrate material layer. According to said patent, two output ends are necessary, detection will only start when an external force is applied, and the detected values must be different. The present patent provides detection without a force being applied. More importantly, for the same sensor, detection is possible even if the values are the same. In the absence of application of a force, stress or action, information can also be read. The problem that the same detected values cannot be read has been resolved. At the same time, two output ends can be reduced to one. Furthermore, wireless transmission is also possible such that the control box can save more power.

### SUMMARY OF THE INVENTION

In addition to the preferred examples or embodiments disclosed below, the present invention can be implemented or otherwise carried out in various ways. Therefore, it is understood that the present invention is not to be construed as limited by the details of configuration and the layout of components described in the following description or shown in the drawings. If there is only one embodiment, as described herein, the claims should not be limited to that embodiment. In addition, the claims are not strictly limited unless there is clear and convincing evidence of exclusion, restriction or disclaimer. The objective of the present invention is to read any external environmental changes and internal information, which can be read regardless of whether it is affected by external forces with fewer wires. The sensing system of the present invention can be applied to fabrics, leather, garments, bed sheets, seats and the like. They have fewer connectors so that the user can be more comfortable; they can also detect responses of the external environment. Any abnormality of sensing components can be immediately notified to the user, and deficiencies of the equipment and points for attention can be sensed. Human or animal behaviors can be detected, and therefore it can be used as an identity card and in behavioral medicine.

In view of the above shortcomings, an objective of the present invention is to provide a sensing system that can detect and read information even if the values are the same, with or without application of a force, stress or action. It has fewer connectors so that the user feels more comfortable; responses of the external environment can also be detected.

Another objective of the present invention is to provide removal of electrostatic interference and even storage of electricity. Electrostatic effect can also be used to measure physiological signals by the fabric adhering to the body.

Another objective of the present invention is to provide using a pattern or body painting applied to the skin as a transmission wire or an electrode, which can produce an insulation effect or an effect of generating and eliminating static electricity. Our system on the fabric can interact with it. In other words, the two can operate in parallel. This also serves as a back-up. Another objective of the present invention is to provide a method of generating a physiological signal by using a fabric resistance sensor or an impedance plethysmogram sensor.

Another objective of the present invention is to provide generation of electricity by electromagnetic induction on the fabric.

Another objective of the present invention is to provide a transmission wire that can be used as a sensor, can be converted to energy for storage, and can also be used as an antenna for wireless transmission.

Another objective of the present invention is to provide a system through which the measurement of an impedance between electrode and body surface is performed by measuring an impedance between a single electrode and the body surface.

Another objective of the present invention is to provide a system that can be used as an identification of human or animal, which can replace an identity card. The above description is only a brief overview of the technical aspects of the present invention. In order to further understand these and other objectives and technical aspects of the present invention, and to implement the present invention based on the description, we provide the following embodiments and drawings:

### BRIEF DESCRIPTION OF THE DRAWINGS

FIG. 1 shows a first configuration of the present invention (in parallel);
FIG. 2 shows a second configuration of the present invention (in series);
FIG. 3 shows a selector implemented by an LC and a band pass filter connected in series;
FIG. 4 shows a frequency response diagram of three identical thermistors;
FIG. 5 shows the use of Zener diodes as selectors;
FIG. 6 shows the effect of 4 selectors;
FIG. 7-1 shows a response diagram of a resistance sensor;
FIG. 7-2 shows a response diagram when a capacitor is added;
FIG. 8 shows adding a capacitor to a resistance sensor;
FIG. 9-1 shows a fabric inductive pneumograph;
FIG. 9-2 shows another fabric inductive pneumograph;
FIG. 10 shows an embodiment with four sensors;
FIG. 11 shows the lower row of electrodes as a low impedance electrode;
FIG. 12 shows the use of stray capacitance to generate only one physical wire and one connection point;
FIG. 13 shows the use of one more reference electrode in the lower row to obtain a high quality signal;
FIG. 14 shows an electrode in the lower row to be used as the sensing electrode that can be connected to electronic components in series;
FIG. 15 shows an embodiment with multiple sets of sensing electrodes;
FIG. 16 shows an embodiment of electrocardiogram electrodes and electromyogram electrodes sharing the same set of wires;
FIG. 17 shows a schematic diagram of the electric circuit of electrocardiogram electrodes and electromyogram electrodes sharing one wire;
FIG. 18 shows multifunctional detection with different frequencies;
FIG. 19 shows an configuration of a wearable system using optical fibers;
FIG. 20 shows another configuration of a wearable system using optical fibers;
FIG. 21 shows a diagram of the contact between optical fibers to transmit light from an under garment to an outer garment;
FIG. 22 shows a configuration of an electrical signal being transmitted through an optical fiber;
FIG. 23 shows a diagram of implementing ground wire conduction using the human body conductivity;
FIG. 24 shows a diagram of simultaneously starting more than two selectors to pick up sensor signals;

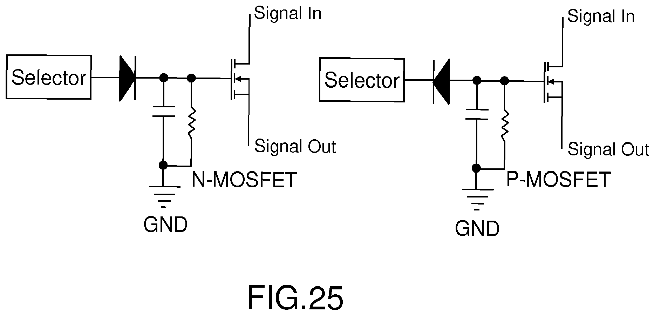

FIG. 25 shows a selector connected to a detection circuit;
FIG. 26 shows a diagram of the electrophysiological signal and the sensor signal simultaneously sharing a set of wires;
FIG. 27 shows a complete configuration diagram of a multifunctional system and backup components;
FIG. 28 shows a diagram of capacitively coupled electrodes used in parallel with conventional electrodes;
FIG. 29 shows a diagram of the effect of electrostatic induction;
FIG. 30 shows a detailed illustration of the effect of electrostatic induction;
FIG. 31-1 shows the effect of electrostatic induction of two layers of fabric;
FIG. 31-2 shows the effect of electrostatic induction of two layers of fabric;
FIG. 31-3 shows the effect of electrostatic induction of two layers of fabric;
FIG. 32-1 shows a configuration of an electrostatic sensor;
FIG. 32-2 shows a detailed illustration of an electrostatic sensor;
FIG. 33 shows a diagram of friction generating static electricity;
FIG. 34 shows a diagram of wind motion generating electricity;
FIG. 35 shows the design of a battery fuel gauge;
FIG. 36-1 shows a top view of a diaper;
FIG. 36-2 shows a side view of a diaper;
FIG. 37 shows a diagram of a capacitor battery;
FIG. 38-1 shows the design of a backup component;
FIG. 38-2 shows the design of a backup component;
FIG. 39 shows a diagram of an electric energy recovery wire;
FIG. 40 shows a transmission wire that can serve as a radio frequency (RF) antenna, a power transmission wire (DC), and a transmission wire for the sensing signals (AC);
FIG. 41 shows a transmission wire that also serves as an antenna, transmitting from under garment to the outer garment;
FIG. 42 shows a diagram of generating electricity by the oscillation of a magnetic material;
FIG. 43 shows a diagram of generating electricity by the oscillation of another magnetic material.

### DETAILED DESCRIPTION

### Part 1: Reduction of connection wires

The existing SPI or I2C interface is designed to meet this one-wire requirement. However, both SPI and I2C require a micro-controller and an appropriate power supply. To make the wearer feel comfortable and move without interference, the sensor must be very small, the connection wire must be very few, and it is difficult to have a power supply. With so many limitations, it is difficult to connect multiple sensors using known SPI or I2C. The present invention discloses a circuit architecture, in which a micro-controller can connect multiple (analog) sensors at the same time by using two wires, and can detect the responses of the sensors respectively.

For example, the sensor suitable for use in the following embodiments may be a microphone (for picking up cardiopulmonary sounds or voices), a physiological electrode, a hygrometer, a thermistor, a resistor or a capacitive strain gauge, a photoresistor, a photodiode (with optical filters of different wavelengths, becoming UV light, visible light or infrared light sensors to sense the brightness of the environment), barometric sensors, gas sensors (alcohol, carbon monoxide or carbon dioxide sensors).

In addition, the sensors described in the following embodiments are also applicable to various actuators, such as vibration motors, LEDs, horns, buzzers, cuffs containing an airbag, electrode resuscitation devices or electrodes used in transcutaneous nerve electric stimulators (TENS) etc.

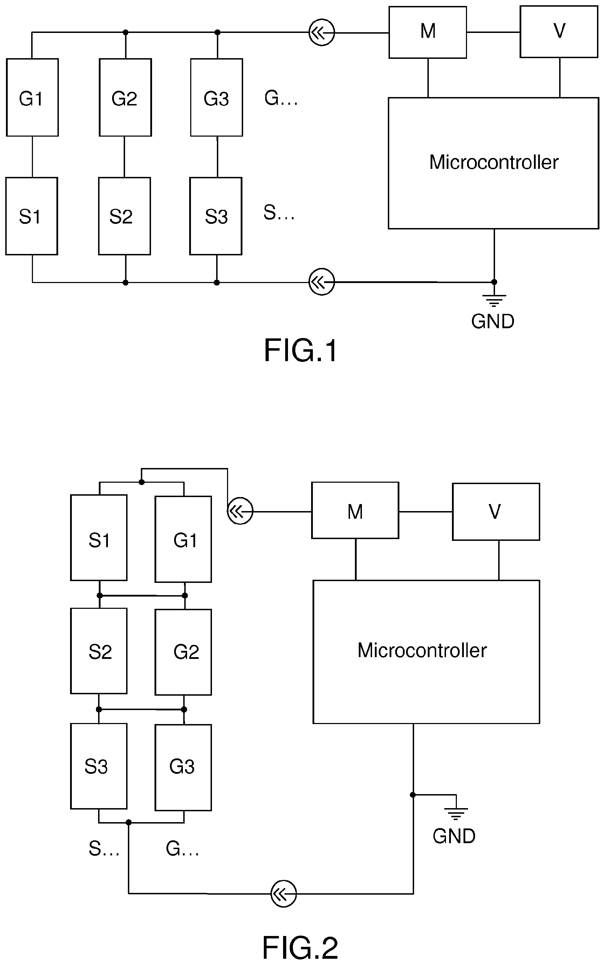

A first configuration of the present invention is shown in FIG. 1, where G represents a selector. Each selector G has different switch-on and switch-off conditions, so that a main frequency generator V can selectively switch on certain selectors G but switch off other selectors G by sending out signals of different characteristics, such as different frequencies or different biases. S represents a sensor (such as a thermistor or strain gauge). Each sensor is connected in series to a selector, and then the sensors are connected in parallel and are connected to two wires. One of the two wires is a shared ground wire (GND) and the other is a signal line. A signal from the main frequency generator V (a voltage source that can change frequency or bias) is set by a microcontroller, and reaches an inlet of each selector via a voltameter M (measuring a current). Each selector G will be switched on or off subject to the signal characteristics of the main frequency generator V to let the voltameter M capture a specific sensor signal. The signal is then digitized and transmitted to the microcontroller.

One of the selectors (such as G3) can also be omitted in FIG. 1, allowing one sensor (such as S3) to be directly connected to the voltameter M, so that S3 can still be sensed by the voltameter M when the frequency generator is set such that the other selectors (G1 and G2) are in an off state. When any other selector (such as G1) is on, the voltameter M will obtain S1+S2. As S1 is known, S2 can also be obtained.

A second configuration of the present invention is shown in FIG. 2, where G represents a selector. Each selector G has different switch-on and switch-off conditions, so that a main frequency generator V can selectively switch on certain selectors G but switch off other selectors G by sending out signals of different characteristics, such as different frequencies or different biases. S represents a sensor (such as a thermistor or strain gauge). Each sensor is connected in parallel to a selector, and then the sensors are connected in series and are connected to two wires. One of the two wires is a shared ground wire (GND) and the other is a signal line. A signal from the main frequency generator V (a voltage source that can change frequency or bias) is set by a microcontroller, and reaches an inlet of each selector via a voltameter M (measuring a voltage). Each selector G will be switched on or off subject to the signal characteristics of the main frequency generator V to let the voltameter M capture a specific sensor signal. The signal is then digitized and transmitted to the microcontroller.

One of the selectors (such as G3) can also be omitted in FIG. 2, allowing one sensor (such as S3) to directly be connected to the voltameter M, so that S3 can still be sensed by the voltameter M when the frequency generator is set such that the other selectors (G1 and G2) are in an on state. As mentioned above, S1 and S2 can also be obtained.

Those from the previous patents US8193465B2, US20100170704A1 and US8331097B2 can all be used as selectors, but under a condition that an external force must be applied in order to start the detection of signals or to provide therapeutic heating.

First Embodiment: the selectors are implemented by LC band pass filters.

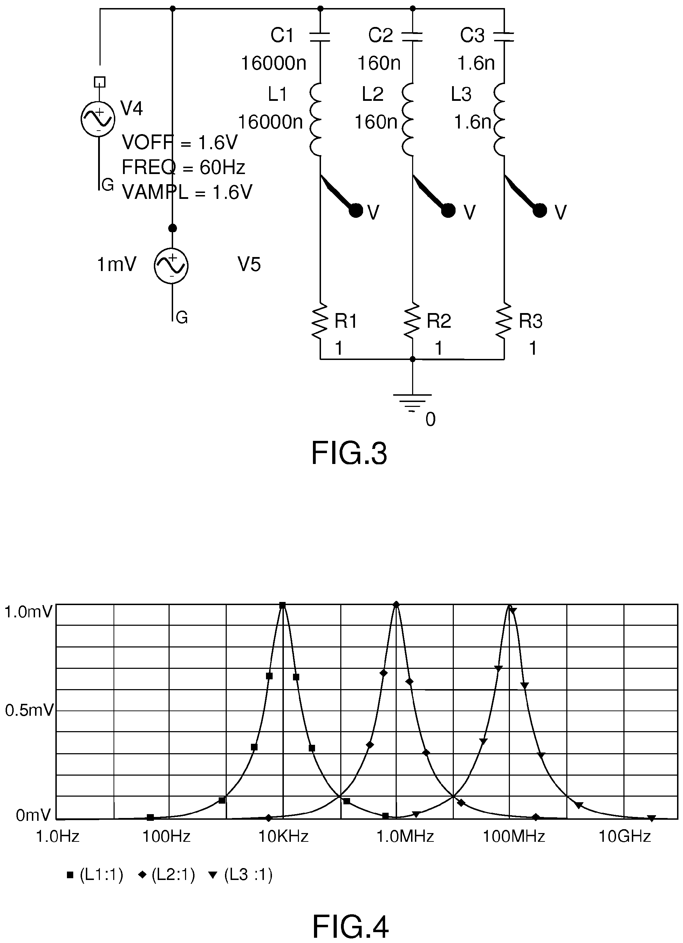

As shown in FIG. 3, a band pass filter with a center frequency of 100 Hz is used as selector G1, a band pass filter of 10K Hz is used as selector G2, and a band pass filter of 1 M Hz is used as selector G3. The sensors S1, S2, and S3 are thermistors with the same characteristics (1 Ω at room temperature), which are installed at the left armpit, the right armpit and the collar of the user, respectively, to sense the temperatures of the left and right armpits and the ambient temperature. The band pass filter is implemented by an inductor and a capacitor connected in series, and its frequency-impedance is shown in FIG. 4.

The frequency generator V is a constant voltage source with adjustable frequency. When the frequency generator V generates a frequency of 100 Hz, the impedance of selector G1 approaches zero, while selectors G2 and G3 approach 200K Ω. If sensors S1, S2 and S3 are 1 Ω thermistors, the series impedance of G1 & S1 is 1,001 Ω, while the series impedance of G2 & S2 and G3 & S3 is 200.001K Ω. In other words, the interference generated by the two groups of sensors and selectors, G2 & S2 and G3 & S3, is one percent of the sensing signal of G1 & S1. Similarly, when the frequency generator V generates a frequency of 10K Hz, the main sensing signals come from G2 & S2. When the frequency generator V generates a frequency of 1 M Hz, the main sensing signals will come from G3 & S3. For applications not requiring relatively high accuracy, interferences caused by the other two groups are negligible, and the results obtained directly at a certain frequency approximate the theoretical value; for applications requiring high accuracy, three measurement results can be obtained using three frequencies, and then more accurate S1, S2, S3 can be obtained by solving simultaneous equations with three variables. The above circuits can be simulated by SPICE (which is widely used in the electronics industry) to get results very close to actual ones to facilitate circuit design.

The selector may be implemented by a ceramic or quartz band pass filter, which is characterized by a narrow frequency band and can accommodate many sensors at the same time.

Or it can be implemented by a reverse-biased Zener diode whose dynamic impedance is negligible.

It is also possible to implement the selector with a constant voltage component such as LM385 or a reed switch or a multiplexer.

Take reed switch for example. A thermistor is placed at an armpit of a side of a top. A magnet is placed on the inside of the sleeve on this side, and a reed switch is placed on the outside of this side. When the arm is in a sagging posture on this side, the temperature of the armpit is close to the core temperature of the body. Meanwhile, the magnet is close to the reed switch to form a connected state. At this time, M can sense the correct body temperature.

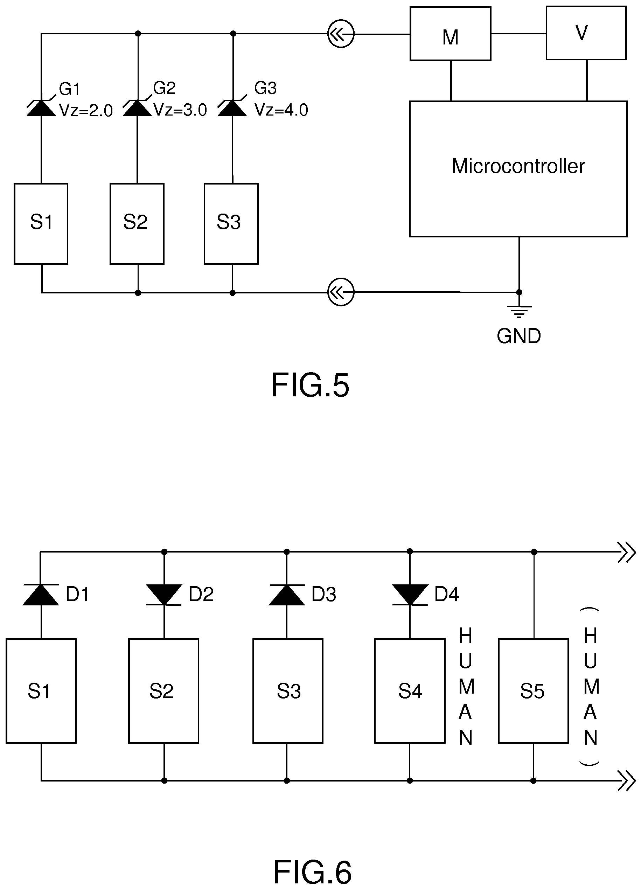

Second Embodiment: As shown in the circuit of FIG. 5, G1, G2, and G3 are diodes having a Zener voltage of 2.0, 3.0, and 4.0, respectively.

Third Embodiment: the selectors are implemented by rectifier diodes in opposite directions

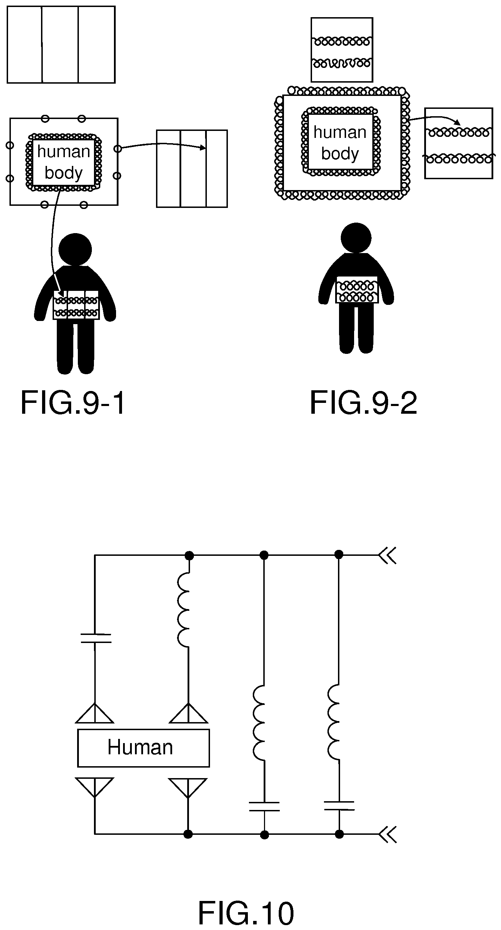

An effect similar to Zener diode effect can also be formed by using different types of diodes having different forward biases as shown in FIG. 09.

S5 is directly connected to the voltameter without a selector therebetween. When the bias and amplitude are less than 0.2V, only S5 will be sensed by the voltameter, while the other sensors are not turned on. Because D1∼D4 are not turned on, the value of S5 is obtained. When the frequency generator gives a bias of +0.3V, S4 plus S5 can be obtained. Similarly, S5 can also be the electrode of an electrocardiogram, electromyogram or electroencephalogram. Because the amplitude of these physiological signals is much less than 0.2 V, D1-D4 will not be turned on and confusion with the signals of other sensors will not occur.

S5 in FIG. 6 may also be a set of conductive fabrics (electrodes) for picking up electrophysiological signals and sharing with other sensors. First a current value of S5 is measured with a zero bias, and then D1 is turned on by adjusting the magnitude and direction of the bias. Similar to the example above, a sum of the currents of S1 and S5 is obtained, and then the current value of S5 is subtracted.

Two conductive fabrics S5 in contact with the human body can also measure breathing (patent US 20130066168 A1). Further, S1 and S2 can be connected to two capacitive sensors to read breathing, heartbeat, posture or movement. The two capacitive sensors can measure the capacitance therebetween using a sinusoidal wave or a pulse wave as described in patents US 8331097 B2, US 7750790 B2, US 20130066168 A1.

The conductive fabric can also be connected in parallel to a resistive sensor, and the sensor does not need to be connected in series to a selector and can be distinguished by DC and AC.

When the capacitance between the conductive fabric and the human body is difficult to read due to sweat, S1/D1 and S2/D2 can be connected in parallel in the present invention. After a slight bias is applied, S1, S2 and the capacitive electrode can be selected. Although there is a bias, it is still predictable and estimable.

If S2 can be an active component such as a microphone, as long as the body impedance between the two electrodes is greater than the output impedance of the microphone, D2 that is originally connected to S2 can be omitted. S2 and the electrodes are connected in parallel, and the wires can still be shared.

When the capacitance between the conductive fabric and the human body is difficult to read due to sweat, an impedance plethysmogram can be read instead in the present invention, and the breathing, body motion and the like can still be obtained. In this case, a resistance (R, real part) and an inductive reactance (X, imaginary part) are obtained at the same time. It can also be used to estimate body fat or body water, that is, to achieve multi-function and multi-purpose, such as J Intern Med Taiwain2012; 23: 245-253. Alternatively, a resistance value can be measured using a DC current, and the skin resistance or sweat can be measured.

In FIG. 6, S1, S2, S3, S4 can be regarded as a set of electrodes attached to specific parts of the body, for example, a set of impedance plethysmography electrodes which are in contact with the human body (the electrodes are made of conductive materials and fixed on the fabric, for example, silver fabric). S1, S2, S3, S4 can be regarded as variable resistors. A weak high frequency (about 50 KHz) current is inputted from a control box, and the voltage at both ends will change with breathing or motion. Thus, it can be used as a sensor for breathing or motion. With 4 electrodes, blood pressure (Sensors 2014, 14(8), 14858-14872) can also be measured. As described above, the frequency generator of the control box can output a bias of a specific direction and magnitude, and one of S1, S2, S3 and S4 can be turned on separately without interfering with each other. This configuration can connect an electrode in parallel that measures an electrophysiological signal, and interference with S 1, S2, S3, S4 will not occur.

The above electrodes can also be used as ECG electrodes to achieve multi-function. For example, as shown in prior patents: direct current resistance (sweat degree) is measured when initiating, and capacitance is measured in the case of an alternating current. If it is wet enough, a traditional ECG circuit is used. If it is too dry, a capacitively coupled electrocardiogram circuit is substituted, and a separate feedback electrode is started. The feedback electrode can be connected in series to a capacitor.

To distinguish between two sensors (also electrodes), one can be added with a capacitor (S2+cap), the other one is without a capacitor (S1). Thus, S1 is measured in the case of DC and S2 is measured in the case of AC.

As shown in FIG. 09, C4/R1 or C5/R2 can be selected by applying different bias directions. Then, by applying DC or AC, R is measured separately or RC is connected in parallel.

Impedance plethysmogram is a method of generating a physiological signal using a fabric resistance sensor or an impedance plethysmogram sensor, and it is characterized in that it comprises at least one fabric, at least one conductive area is disposed on said fabric, and a signal circuit is provided. The conductive fabric forms a resistance or impedance with the human body, and the fabric resistance sensor or the impedance plethysmogram sensor is connected in series or in parallel to a resistor R, a capacitor C, an inductor L, an operational amplifier, a diode, a Schmitt trigger, a CMOS field effect crystal, a transistor, or an integrated circuit to form a charge/discharge circuit to change the frequency, period, voltage or current. When there is pressure, pulling force, torque or tension between the human body and the fabric, the circuit sends a signal. The system receives a change in a value of the resistance sensor or the impedance plethysmogram sensor between the conductive fabric and the human body, and said change is represented by a change of frequency, voltage or current. At least one of the information of physiology, medium (sweat, blood, lip balm), posture changes, medium changes between the human body and the fabric, or the force received is analyzed through the change in frequency, voltage or current.

The method is exactly the same as the prior capacitor patent US 20130066168 A1, except that the former is to measure a capacitance, and now the impedance between the conductive fabric and the human body is measured, so the details are the same as in the above patent and will not be described herein.

In the above system, a switch is connected to the conductive fabric or the switch itself is a resistance sensor or an impedance plethysmogram sensor.

In the above system, changes in physiological state, posture and force can be measured by changes in frequency and voltage-current curves.

In the above system, there are at least two said conductive fabrics, one of which is a reference conductive area, and said reference conductive area is grounded.

In the above system, the system can be used to detect posture, and can also be used to detect physiological information such as heartbeat, breathing, humidity, swallowing, sweating, body temperature and cough.

The above system can also be used for positioning, that is, to know where a physiological signal is generated in the body.

In the above system, the conductive area can be further used as a physiological signal sensor, that is, an electrode used to detect heartbeat, respiration, brain wave, electromyogram, electrocardiogram, body fat content or sweat; or the conductive fabric is used as an treatment electrode to produce transcutaneous nerve electrical stimulation, heat or cooling.

In the above system, the conductive fabric is designed on garments, hats, socks, shoes, bed sheets, gloves, steering wheels, crutches, table cloths, carpets, or prostheses.

The above system can also be used to detect changes in resistance or impedance caused by sweat, wounds, sweating, medicine application and powder makeup.

The above system can also detect joint angle, angular velocity, and angular acceleration.

The above system can also detect a person's position, speed, acceleration or distance traveled.

The above method can also be used to detect gait, gait stability, fall, sleep activity map or movement.

The conductive fabric may not be in direct contact with the human body, and a fabric, a rubber, a plastic film, a waterproof fabric, a coated fabric and printed material may be placed between the conductive fabric and the human body.

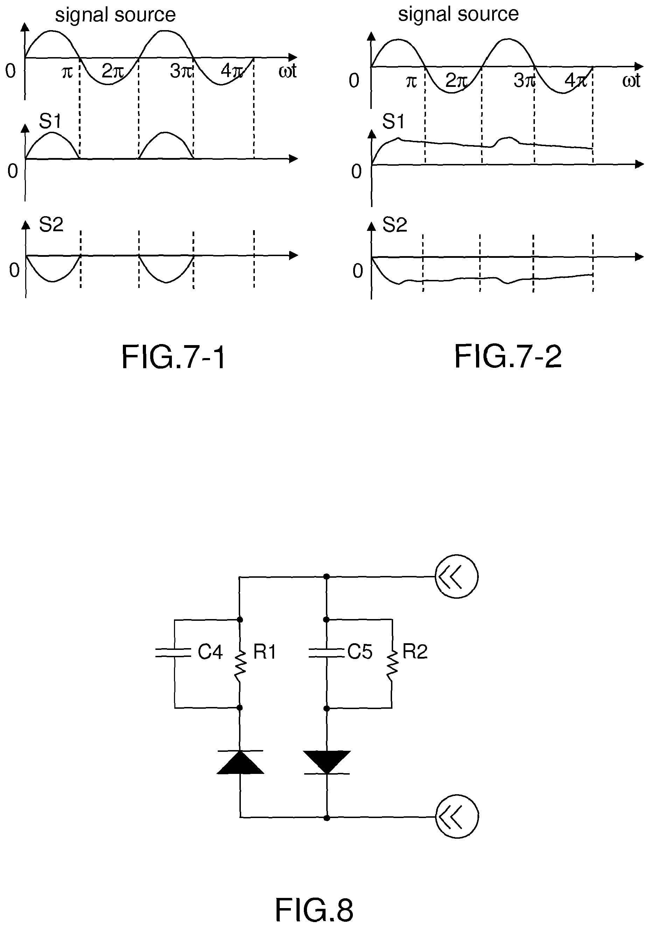

As shown in FIG. 6, if the sensors are all pure resistors, the current waveform is a rectified sine wave. Thus, it is a sine wave for a semi period and zero for the next semi period (FIG. 7-1). In the case where only simple circuit and signal processing techniques can be used, it is not easy to obtain the true impedance for this type of waveform. In order to obtain more accurate results, a capacitor can be added next to the resistive sensor, and a simple circuit and a signal processing method can be used to obtain more accurate results, as shown in FIG. 8. Thus, it is not necessary to capture a signal at a peak of the sine wave, and a result with minimal deviation can still be obtained. As shown in (FIG. 7-2), a simple circuit and a signal processing method can be used to obtain relatively accurate results.

Meanwhile, a fabric inductive pneumograph can be used to measure breathing or body motion. It can also be an antenna or an electromagnetic wave or magnetic field sensor, so that posture, stature, whether a weight is being carried, and how much weight in the handbag can be measured. Similarly, the inductive pneumograph can be connected in parallel to a thermistor, so that body temperature or ambient temperature can be measured at the same time.

The human body can be wrapped with multiple inductive pneumograph sensors, which can not only read the breath, but also read the changes in the contraction at this place. There is also mutual inductance between inductive pneumograph sensors, which can also be used to measure the mutual motion between various parts of the body, as shown in FIG. (9-1) and FIG. (9-2).

As shown in FIG. 10, an inductive pneumograph sensor (the advantage is that it does not directly touch the human body) can also be connected in parallel. If the user is sensitive to the electrode, the respiratory signal can be read by the inductive pneumograph sensor. Considering that the inductive pneumograph sensor itself is an inductor, the DC impedance is very low, if it is directly connected with other electrodes or sensors, it may cause interference. A capacitor series inductive pneumograph sensor can be used in the present invention, and the frequency generator measures the inductive pneumograph via LC resonance frequency of the group, which will not cause interference. As shown in FIG. (10), the inductance of the two sets of LC on the right side is an inductive pneumograph sensor. If a capacitor is connected in series, a specific resonant frequency can be obtained. As long as the frequency band of the electrode is different with that of the above figure, it can be detected separately without worry about the interference.

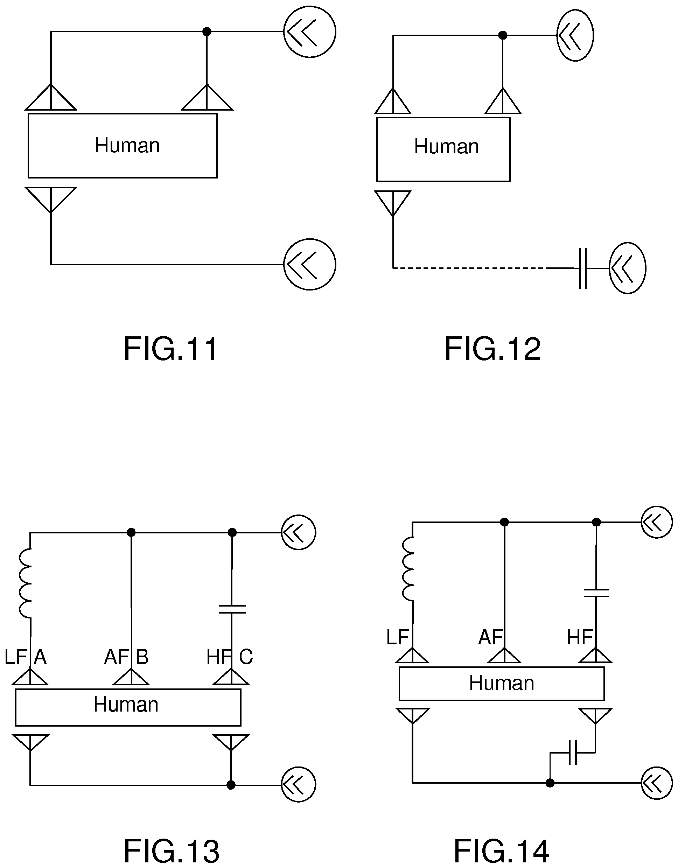

In FIG. 11, the lower row of electrodes can be designed as a low impedance electrode to serve as a reference electrode. The lower row of electrodes can also be sensing electrodes. The upper row of electrodes serves as sensing electrodes, and their impedance value varies depending on the respiratory motion or other physiological conditions where it is located. A reference electrode (low-impedance electrode) can be shared, and the signals of the two sets of electrodes connected in parallel (for example, chest and abdominal breathing sum) can be read but can't be separated.

The present invention can also use the stray capacitance between the control box and the human body to connect the reference electrodes, that is, one of the contacts can be omitted, so that there are only one physical wire and one connection point, as shown in FIG. 12.

As shown in FIG. 13, a reference electrode in the lower row is sufficient. When there is one more reference electrode, as long as one electrode has the same function as the conventional electrode (the impedance is the same low), that is, one more guarantee, as a result, a high quality signal can be obtained. The sensing electrodes can achieve frequency separation by a L or a C in series. L rejects high frequencies to pass through low frequencies (LF), C rejects low frequencies to pass through high frequencies (HF), instead, all frequencies are conductive (AF) without LC, thus three frequency bands are separated. The actual measurement method is: give low frequency (LF) signal to get A+B, give intermediate frequency (IF) signal to get B, give low frequency (HF) signal to get C+B.

If there is an electrode in the lower row to be used as the sensing electrode instead of as a reference electrode, electronic components (such as capacitors, inductors, diodes, LC, etc.) can be connected in series, and the potential can be separated from the reference potential. The capacitor is as an example in FIG. 14. Similarly, other parts can be connected in series, as mentioned above, and so on.

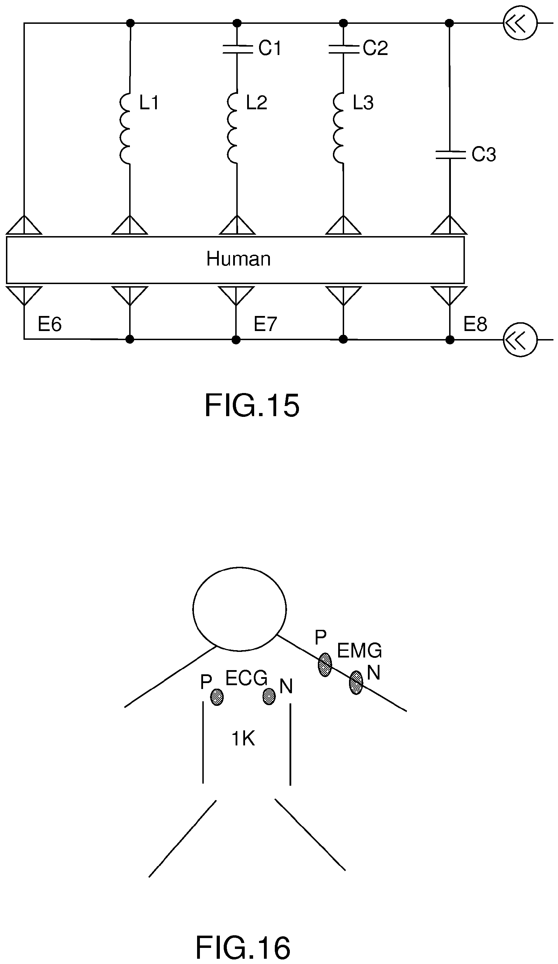

FIG. 15 is an LC band pass filter that can accommodate multiple sets of sensing electrodes. As described above, one or more of the lower electrodes E6 to E10 may be omitted depending on the application.

The L or C filter, plus two diodes in different directions, can accommodate multiple sets of sensing electrodes. As long as the bias is applied in different directions, one set can be turned on and the other set can be turned off. Similarly, a Zener diode of different reverse collapse voltages or a rectifier diode of different cut-in voltages can be used to parallel multiple sets of sensing electrodes without interfering with each other.

The conductive fabric is used as an electrode to pick up the physiological electrical signal, and the different frequencies of the signal itself can be used to realize the simultaneous transmission of multiple signals in one line. For example, an electrocardiogram electrode can share the same set of wires with an EMG, as shown in FIG. 16.

For example, an electrocardiogram electrode includes two types: a dry electrode and a capacitor electrode. The dry electrode can read the physiological electrical signal via a cotton conductive fabric with high water absorption. Carbon fiber and conductive silicone fabrics are suitable for capacitive electrodes. On the various parts of the body, the degree of sweating is different and the skin quality is different, so the area of the dry electrode is required to be different. The impedance of the finger skin is low, and the skin impedance of the baby is low, so the electrode area can be reduced. Since the measured skin impedance is 10MΩ via the stainless steel wire and is 2 MΩ when the skin is very wet, the ECG can be read when the impedance of dry electrode ECG is less than 5 MΩ. According to this characteristics, the stainless steel wire can be used as the transmission wire of the ECG. No coating is required and it will not interfere with the reading. If the dry electrode needs to maintain water saturation for a long time in actual human body measurement, it can be coated with water-saturated materials such as PVA or crystal mud, or waterproof materials such as PU, which are added to the dry electrode to ensure the moisture release within two days so as to realize that the impedance of the surface conductive fabric is within 5 MΩ.

The previous US 20140343392 A1 fabric has a mesh fabric on the periphery of the electrode, as shown in FIG. 7 (h), which has a filtering function. Now the mesh fabric is replaced by a ring of elastic band, the effect is the same. When the silver fabric or conductive fabric with filtering function moves on the elastic band, you can also read ECG, EMG, brain waves, etc.

The electrocardiogram electrodes are attached to the sides of the body, and the EMG electrodes are placed on the left upper arm. In order to reduce the interactive interference of the two sets of electrophysiological signals, in the present invention, a resistor can be connected at one end of the electrode in series and then connected the common wire. The signal is transmitted to the input (P and N) of the positive and negative terminals of the instrumentation amplifier on the control box, and then two band pass filters of different frequencies are used. The ECG frequency band is 0.6-40 Hz, and the EMG frequency band is 50-300 Hz, which can be obtained separately.

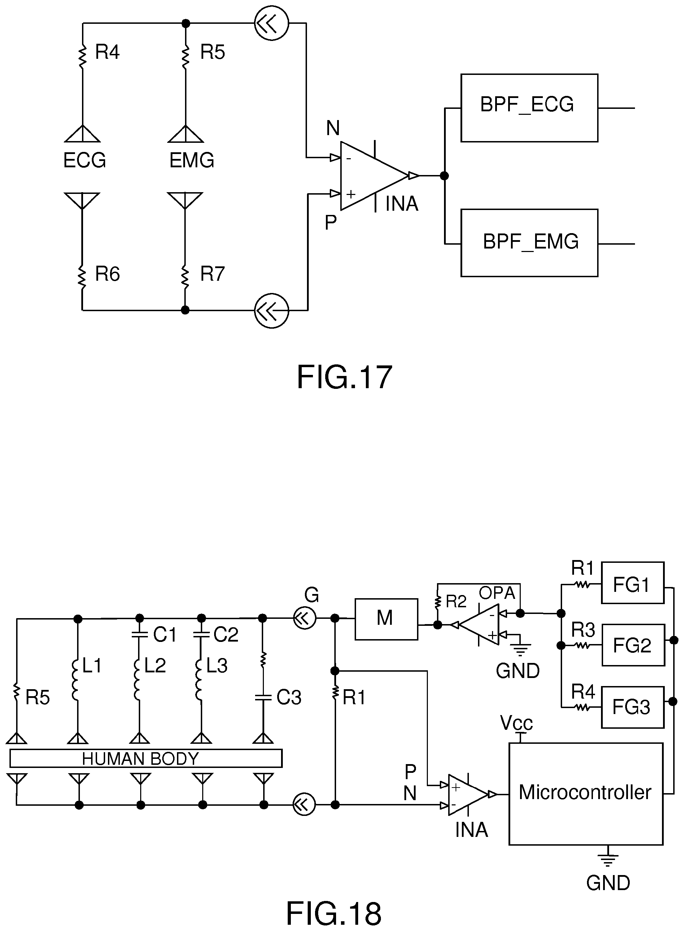

Considering that the output impedance of the electrocardiogram or electromyogram signal obtained by the electrodes at different positions may be quite different, R4∼R7 in FIG. 17 can be selectively omitted, which makes the circuit simpler.

As shown in FIG. 17, the electrode connects resistance in series, and the present invention generalizes this principle. When electrode connects capacitor or inductance in series, the electrode not only can pick up the electrophysiological signal (ECG, EMG or EEG), but also can be used to input current into the body to measure pulse wave via the blood vessel volume change diagram, or measure body motion or breathing, sweat on the skin surface, or body composition (such as body fat and moisture) via capacitive sensors. As shown in FIG. (18), when each electrode is used to input current into the body, the signals with the lowest frequency can pass L1 (for example, 10 Hz), and the signals with the highest frequency can pass C3 (for example, 30 M Hz). Appropriate capacitance inductance values are set to cause the resonance frequencies of L2/C1 and L3/C2 to be 1K and 100K respectively. Then, the frequency generators of different frequencies mentioned above are input from G into the left R, L, C and electrodes respectively, the sensing values of each set of electrodes can be gotten. In general, the body impedance of the electrode in the figure below is about several hundred of Ohms, as long as the impedance of the LC connected to it reaches thousands or even tens of thousands of ohms (far more than hundreds of ohms) at its set frequency, the interactive interference will be low enough. Conversely, when these electrodes are used as electrodes for sensing physiological signals, the physiological frequency generators connected to the R, L, C and electrodes face the input impedance of the control box (represented by Ri). If not intentionally placed a low-resistance resistor, usually Ri will cause the input impedance of the instrumentation amplifier up to several MΩ, which is much larger than the impedance of the human body and the impedance of L, C at the above frequencies, so L1∼L3 or even C1∼C3 can be regarded as low-impedance conductor, and will not constitute an obstacle to the transmission of physiological signals.

The fabric electrode contacting with the human body can be regarded as equivalent to a resistor and a capacitor in parallel. When the skin is dry, the contact resistance is large, but its capacitance still exists. When the skin is dry, it is difficult to obtain a good signal with a conventional electrocardiogram amplifying circuit, at this time, it is possible to switch to a capacitively coupled electrocardiogram circuit which will be described later.

The above wires may also be optical fibers, bare wires, insulated wires, and the transmission wires may be printed circuits, conductive inks, optical fibers, conductive films, conductive fibers, conductive silicone, conductive rubber, and the like.

The aforementioned transmission wires and various types of sensors can also be replaced by optical fibers and optical fiber sensors, which have the advantages of not only being less susceptible to radio wave interference, but also can easily achieve multi-task simultaneous transmission with the same optical fiber, and the optical fiber itself can simultaneously sever as a sensor. If the above transmission wire is replaced by an optical fiber, the transmitted signal between the control box and the fabric can be easily realized by one contact point. For example, there are several fibers fused together on the fabric, as shown in FIG. (19), the sensor ends are emitting different colored lights (R, G, B) via LED, which are transmitted to the control box through a connector. The control box has beam splitter of the wavelength selective optical coating to send the different colored lights to different photodiodes for conversion to electrical signals for subsequent processing by the circuit in the control box.

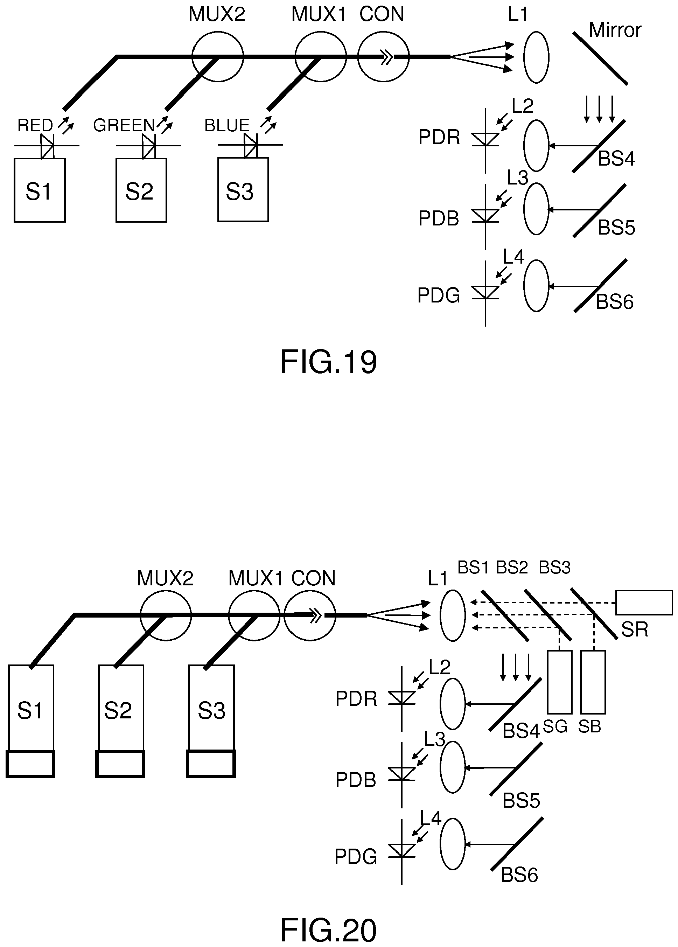

FIG. 19 is an architectural diagram of an optical fiber applied to a wearable system. With different wavelengths, multiple sensors can be connected on the same fiber in the present invention. In the figure, S1, S2 and S3 are three sensors, which are respectively connected with LED of different colored lights. They emit light into the fiber and they are connected to the control box through a single point connector (CON). Red, blue and green lights are input into the different photosensitive diodes (PDR, PDB, PDG) by a beam splitter (BS) and a convex lens (L2, L3, L4) coated with a band pass filter film

For example, the fabric end does not have a light-emitting component, but the light is completely emitted by the control box. It enters into the optical fiber via the beam splitter, and the filter is placed inside the fabric, so that different colored lights enter different sensors. After the light is modulated by the sensor, it will be reflected back to the fiber, and then return to the control box for photoelectric conversion into electrical signals.

FIG. 20 is an architectural diagram of an optical fiber applied to a wearable system. With different wavelengths, multiple sensors can be connected on the same fiber in the present invention. The control box is on the right side of the CON. The red light source (SR), the blue light source (SB) and the green light source (SG) are mixed by a beam splitter (BS) and input into the fiber through a convex lens. In the figure, S1, S2 and S3 are three sensors. A band pass filter with different light colors is between the fiber and the sensor, and only allows specific light waves to pass through. For example, in the above figure, S1 is connected to red light filter, S2 is connected to the blue light filter, and S3 is connected to the green light filter. The light wave of a specific wavelength enters the sensor and is modulated by the sensor, and then reflected by the mirror below the sensor back to the optical fiber, and further transmitted back to control box by optical fiber via the connector (CON) through the multiplexer MUX1 and MUX2 together with other color lights. After passing the convex lens, it is then reflected by BS1 to B4, B5 and B6, respectively, and passes through L2, L3 and L4 coated with a band pass filter to select red, blue and green light, respectively. With this structure, S1, S2, S3 can share one optical fiber, and only one contact is required between the control box and the fabric.

The optical fiber itself can also be a sensor. Take S1 in the figure as an example. If S1 is a piece of optical fiber and there is a mirror at the end, when S1 is pulled, pressed or twisted, its structure will change and the transmission loss will increase, the magnitude of the force is known thereby.

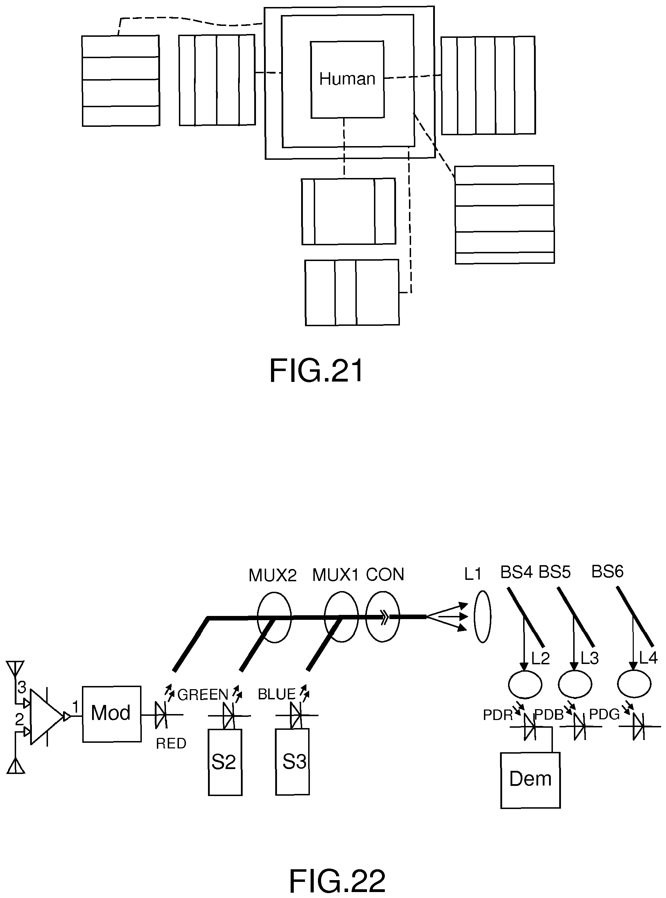

The optical fiber can be used as the transmission wire and a sensor. In addition, it can also be used as a spare wire, and the switching method is as follows: the optical fiber is very thin and soft, which is very suitable for application on fabric. Therefore, it can simulate the the above the example to arrange another piece of optical fiber for backup on the side of the main transmission wire or optical fiber. The MUX1 and MUX2 in the above figure may choose a simple two-beam superposition, a mechanically-operated single-pole double throw switch (which can be switched by motor or manual), or a photoelectric switch (OE switch) which is electrically switched and contains photoelectric crystal. To switch the photoelectric switch, in addition to the directly electrical driving on the control box, a light can be transmitted from the optical fiber to the photoelectric switch, and then the filter is used to select a specific photoelectric switch as the above example. Next, the light is converted into power by a photosensitive diode to drive the photoelectric switch for switching. The optical fiber is in contact with the fiber to conduct light, form the horizontal line to vertical line, from the underwear to the outerwear (as shown in FIG. 21).

Theoretically, the optical fiber can restrain the light from leaking out, but when the optical fiber is pressed by an external force, its structure changes, as a result, it is inevitable that light is leaked out at the pressed position. According to the characteristics, the signals can be transmitted from fabric to other places (such as beds or chairs) in the present invention, without having to transmit signals from the original optical fiber connectors or electrical connectors. As simulated the above example (the wires are criss-crossed), the optical fiber can also select a specific part to transmit signals.

Optical fiber can be used for signal transmission (e.g. ECG) or as an optical fiber sensor (sensing force)

For some signals which are originally electrical signals and are difficult to be directly captured by the light, such as an electrocardiogram, the electrical signal can still be converted into an optical signal through a simple electro-optical conversion circuit and then transmitted. Because light can be easily separated by different light colors, it is easier to transmit multiple sets of signals on the same optical fiber. Similarly, the transmission wire can also be used as signal transmission and as a sensor (sensing force), and can also be used as spare wire.

As shown in FIG. 22, two electrodes that sense physiological electrical signals (ECG, EEG, or EMG) are at the leftmost end. The common mode noise is removed by the instrumentation amplifier to obtain the physiological electrical signal, and then is treated by the modulation circuit (Modulator, Mod, can be the known FM, AM or PWM modes). It is converted into light by an EO converter (represented by LED), and sent to the PDR of the control box for receiving. It then is subjected to a demodulation circuit (Demodulator, Dem) to restore to the original physiological electrical signal. This can ensure that the physiological telecommunication can be transmitted through the optical fiber. Even if the optical fiber is used as the sensor at the same time, as long as the sensed signal frequency and the modulation frequency are different, the physiological electrical signal and the sensing signal can be independently detected, and will not be interactively interfered. If the circuit is to be more simplified, it is also possible to directly transmit the physiological electrical signal via the optical fiber without using Mod and Dem, but at this time, it may be interfered by breathing or body motion. However, at this time, the physiological signal and the respiratory or body motion signal can still be separated by the digital signal processing method, for example, using the known ECG-derived respiration signal technology to get the breathing signal. In general, the amplitude of the electrophysiological signal is relatively stable, and it rarely changes greatly within one minute. The amplitude change in a short time can be attributed to factors (such as breathing or body motion) applied to the optical fiber, that is, it can be gotten via the known demodulation technology.

For example, optical fiber is used to detect the fabric damage. Currently, there is a type of indoor lighting optical fiber commercially available, and it is designed to deliberately leak a small part of the light from the side. If this optical fiber is used, one inside and outside the fabric serves as the receiving terminal, one inside and outside the fabric serves as the transmitting terminal. When the ambient brightness is not strong, it can be used to detect the damage of the fabric. When the fabric is damaged more seriously, the light energy obtained at the receiving terminal is stronger.

Fourth Embodiment: Comparing DC, AC and pulse signals as a selector

| | Advantages | Disadvantage s |
|---|---|---|
| DC | Low power, low cost elements and circuits, ECG, thermistor, humidity, etc. | Baseline DC drift and aging can't be rejected; |
| | No electromagnetic interference to the human body | Possibly be interfered by RF electromagnetics, but can be stopped by the CMCC. |
| | It can sense the EMI by cellular phone and quantify the power of RF by cell phone, from which we can record and analyze the user's behavior pattern and the position (such as the heart or waist) for him/her to fix the cell on the body. DC can be used to verify the entire system. The DC subsystem can be used to shelf the fabric on sale or in laundry. Digital circuit on fabric may save power saving. | |
| AC | Lock-in amplifier can be applied, high signal-to-noise ratio | Complex analog CKT, high energy consumption |
| Pulse | High speed, low power consumption of analog circuits | Requires high-speed computing capacity and firmware; high power consumption of microprocessors |

Fifth Embodiment: Conducting ground conduction with human body conductivity

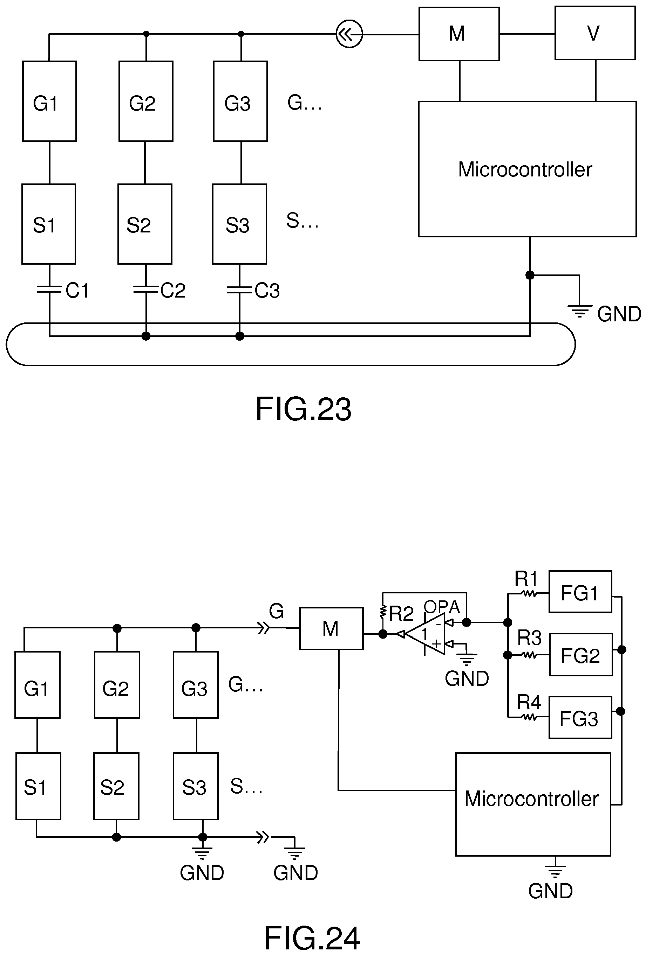

In general, a connection wire in the circuit is defined as a ground wire (Ground, abbreviated as GND). If the foregoing embodiment is applied to a wearable device, the human body can also be used as a ground wire. For high-frequency (frequency greater than 20K Hz) current, if it is less than 20u amp, it will not harm the human body and will not make people feel. The conductive properties of the skin can be represented by a parallel connection of a resistor and a capacitor. The higher the frequency, the lower the impedance of the skin, which is more favorable for the current to flow into the human body. In this embodiment, a small piece of conductive fabric about 1 cm² is placed on the fabric to connect with the sensor, and a capacitance (C1, C2, and C3) is formed in the body. After the current passes through the skin and enters the human body, it is conducted by the highly conductive blood vessel to the whole body (the blue line inside the red frame), just like the conductive copper foil of the circuit board. In this way, the conductivity of the human body is utilized, and a connecting wire that must be made on the fabric is omitted. As for the ground wire of the circuit on the control box (in the figure below (as shown in FIG. 23), the GND of the microcontroller), it can be connected to a close-fitting fabric electrode or the GND exposed conductor (such as a metal piece) of the controller to be in contact with a person.

If capacitive sensing is performed, such as respiratory sensing, as shown in the figure above (FIG. 23.), the GND of the microcontroller can also be implemented with stray capacitance. When the control box is close enough to the human body, the stray capacitance between the circuit board and the human body is large enough. When the oscillation frequency of the control box is high enough, one wire and one electrode can be omitted. According to the above description, if the connector is damaged and the ground wire can't be connected in the foregoing embodiment, the present invention can still implement certain functions through the stray capacitance, and can remind the user to take necessary measures, such as when the stray capacitance is not large enough, the user is reminded to buckle another ground electrode to enhance the effect. There is only one electrode's detection signal, and only one electrode is measured. Its signal characteristics can reveal the characteristics between the electrode and the human body. In particular, the impedance between its electrode and the human body is high or low, that is, the impedance value directly read between the single electrode and the human body, which is to use the concept of virtual ground (AC, DC or pulse signals) to understand the dryness/wetness of the electrode, that is, the degree of conductivity. Once the electrodes are selected, the ECG or EMG can be measured by pairing. In particular, dry electrode detection or capacitive electrocardiogram detection can be selected for the electrocardiogram. If the impedance value of the capacitive electrode is lowered, it can be switched to a dry electrode to measure the electrocardiogram, which can reduce one point and become a two-point type to read the electrocardiogram. The effect of the dry electrode can also be enhanced by the electrode effect of the pattern or the painting on the corresponding positions of the body.

Sixth Embodiment: Simultaneously starting more than two selectors to pick up sensor signals

In the above embodiment, only one selector is turned on, and one end of the sensor is grounded. In this embodiment, two or more signal generators (FG) are used to generate independent frequencies. After an adder (using an operational amplifier as the core), the signals of more than two sensors can be simultaneously captured, and the sensor is not necessary to be grounded, as shown in FIG. 24. The analog output of the voltameter can be connected in parallel with the two filters, and selectively connected to the lock-in amplifier. FG1 and FG2 are used as reference frequency sources respectively, and the signals of the two sensors can be picked up at the same time.

Seventh Embodiment: the selector plus the detection circuit and the field effect crystal to pick up the electrophysiological signal of the living body

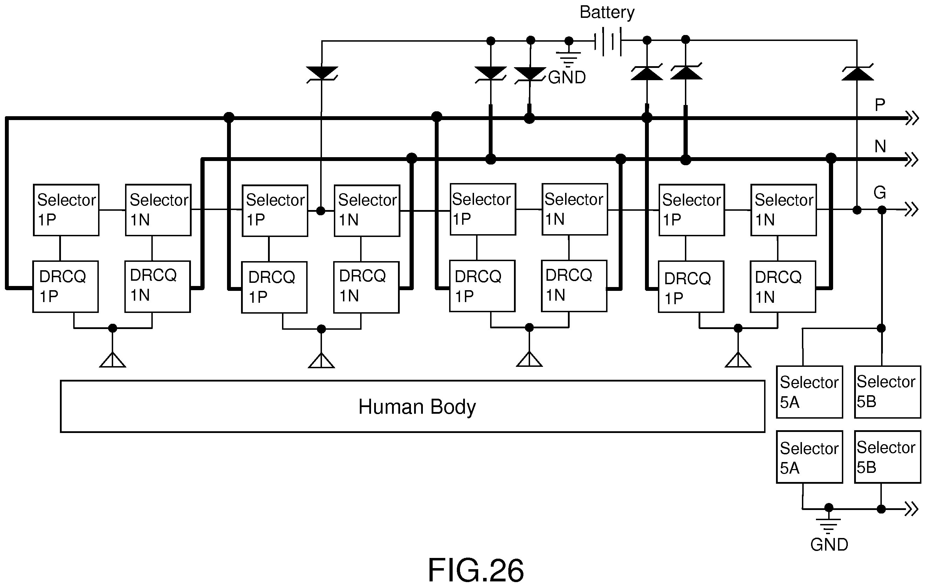

In the foregoing embodiment, the passive sensor is taken as an example. In this embodiment, the function of picking up an electrophysiological signal is further increased, as shown in FIG. (25). The aforementioned selector is connected to a detection circuit, and then connected to a field effect crystal to serve as an analog switch, DRCQ as an representative in FIG. 26. When the selector is activated, C will accumulate charge. When the voltage of C is greater than the gate threshold voltage of Q, Q is turned on. According to the above example, the microcontroller can select two electrodes randomly and transmit the signal to the input terminal of the instrumentation amplifier to obtain the electrophysiological signal.

Each of the electrodes in FIG. 26 is connected to the positive and negative terminals of the instrumentation amplifier. This is a backup connection wire to prevent the open circuit of certain wire. The actual structure diagram is shown in FIG. (27). In this embodiment, a set of wires (P, N, G and GND, four pieces in total) can be simultaneously shared by the electrophysiological signal and the sensor signal, which greatly reduces the connection wire on the fabric. If the seventh embodiment (the ground wire conduction is realized by the human body conductivity) is used, one contact can be omitted.

### Eighth Embodiment

How to verify whether a single electrode is in good contact in this system. You can selectively turn on an electrode in the control box according to the above method, and then measure the impedance by using stray capacitance as GND. Those with high impedance can serve as capacitive sensor to measure breathing or body motion, and those with low impedance is suitable for serving as reference electrode. First select a single electrode and test with the stray capacitance, and then select two electrodes to test. If the noise is too high, the skin may be too dry, in this case, a capacitively coupled electrocardiograph can be considered. If the traditional electrocardiogram effect is not good, the user can be requested to add another connector to connect the feedback electrode to implement the capacitively coupled electrocardiograph.

The capacitively coupled electrode may be the above-mentioned wearable physiological electrode, which is mounted on the fabric in the form of fabric made of conductive yarn. When the skin is very dry, the DC resistance between the electrode and the skin is quite high, but the area is not small, so it is still capacitively coupled with the human body, that is, it can be regarded as a capacitively coupled electrode; the capacitively coupled electrode may also be the aforementioned wearable physiological electrode, which is mounted on a non-closed fitting fabric, a chair or a bed. If sweat does not wet through the closed fitting fabric, it can also be regarded as a capacitively coupled electrode; the capacitively coupled electrode may also be a wearable physiological electrode with a surface-coated or plated insulator, that is, a capacitively coupled electrode.

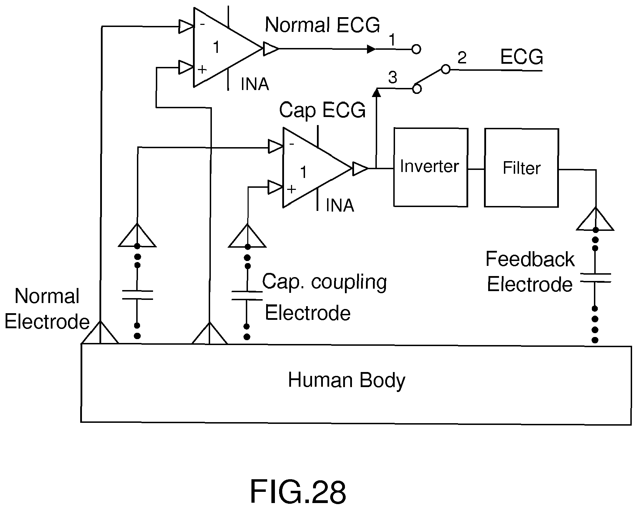

Another time when a capacitively coupled electrocardiograph must be used is that the user is allergic to the silver electrode, resulting in itchy or red skin, in this case, you can use a capacitively coupled electrode to avoid direct contact. The capacitively coupled electrodes can be connected in parallel with conventional electrodes to enhance the effect (FIG. 28). Depending on the humidity of the skin, the methods described above are selected. The electrodes that are in contact with the body, in addition to measuring physiological telecommunications (ECG, EEG, EMG...), can also be used as sweat (in DC) or capacitive sensors (in AC) to measure breathing, body motion, posture and body fat. It had better to measure body fat at the proper time (avoiding after a lot of sweating and drinking plenty of water). In fact, bio-impedance is also measuring the water in the body, either skin is too dry or too wet is not suitable. For measuring body fat, if there is no electrode close to the skin, the application of stray capacitance still can measure body fat, the principle of action is: body impedance measured by the body fat meter usually can be represented by a resistor in parallel with a small capacitor, and such small capacitor is about InF. If the control box can be close to the human body, and its casing is thin enough, so that the stray capacitance between the circuit board and the human body is greater than InF, and the body fat measurement value with less deviation can be measured. In summary, the three functions of measuring body fat, sweat and water in body can be realized at the same time, and whether the electrode in good condition is verified, which save the power. Although it may not be accurate at first, after accumulating a large number of data for a long time, the normal mode can be established, thereby estimating the routine water drinking, sweating and other behaviors.

### Ninth Embodiment: Static electricity elimination

The Electrostatic Discharge (ESD) of human body model (HBM) refers to the accumulation of static electricity on the human body when the human body walks on the ground or due to other factors. When such person touches the electronic parts, the static electricity will burn out the parts.

In addition, the static electricity will also interfere with the signal of the wearable device line, and it is not good for the human body, therefore, there is a need to eliminate static electricity. In addition, static electricity has characteristics that can be utilized: because of the adsorption effect, the human body is a conductor, so the static electricity attracts the insulator to the human body. The materials on the periphery of electrode or sensor, or the materials containing static electricity themselves will be naturally adsorbed to the human body. The adsorption is better than the adhesion to the human body, and the transmitted signal will be more stable. The pattern or body painting applied on skin can create a static effect, and the electrodes on the fabric can interact with it (adsorption effect).

We can use the skin tattoo pattern or painting materials, which are conductive, so they can be used as electrodes, and can be added with static-containing materials to enhance the effect (adsorption effect). Skin tattoo pattern or painting can also be used for signal transmission of line, and it can also be added with anti-static agent to enhance the effect. The non-metal or organic materials can also be used for insulation. Painted materials include water-based (conductive) and non-water-based ones. The advantage of water-based materials is that they can achieve the conduction effect with conductive fabric. The non-water-based materials can be used for insulation effect, and anti-static agents can also be added to enhance the effect.

The shortcoming of pattern is that they are fixed and cannot be easily removed. The painting is easy to apply and convenient to remove.

Only the area where the sensor reads static electricity is kept, and the other unnecessary parts are coated with anti-static agent to prevent the damage of static electricity. In this way, the sensor can become the electrostatic sensor we want. The sensing components themselves can be the fabrics A, B and C shown in the in FIG. (31-2) and (31-3). The difference is that A is all positively and negatively charged arrangement, B is positively charged, and C is either a component of fully negatively charged fabric, or made of plastic, rubber, silicone, film etc. For example, these positively and negatively charge will not move, therefore, if there is a negatively charged fabric on the outer of the fabric, it can attract the positive charge in the air to produce a clean effect.

In the winter, it is often dry, which usually causes static electricity.

After the relative humidity is greater than 55%, the object is hard to accumulate high-voltage electricity, so it is not easy to generate static electricity.

The effect of relative humidity on static electricity is that:
1. Relative humidity affects the conductivity of the body surface and air, thereby affecting the speed at which the accumulated net charge leaves the human body.
2. High relative humidity enhances the adsorption of water on the surface of the object, so that the friction of two different objects does not easily produce a distribution of net charge. The greater the humidity of the fabric, the less easy the electricity is to run. The wetness or dryness can be sensed by detecting the environmental changes in the season based on induction or triboelectric generation. The sensing component can be measured.

Because the advantage of smart fabric is that they are shielded by the electrostatic effect of conductive fabric (wire), and positive charge will disappear. Human being are conductors, and conductive fabric (wire) can prevent electromagnetic interference. When wearing smart fabric, the positions with organs shield by conductive fabric (wire) can be protected from electromagnetic waves, and the human body can prevent electromagnetic interference.

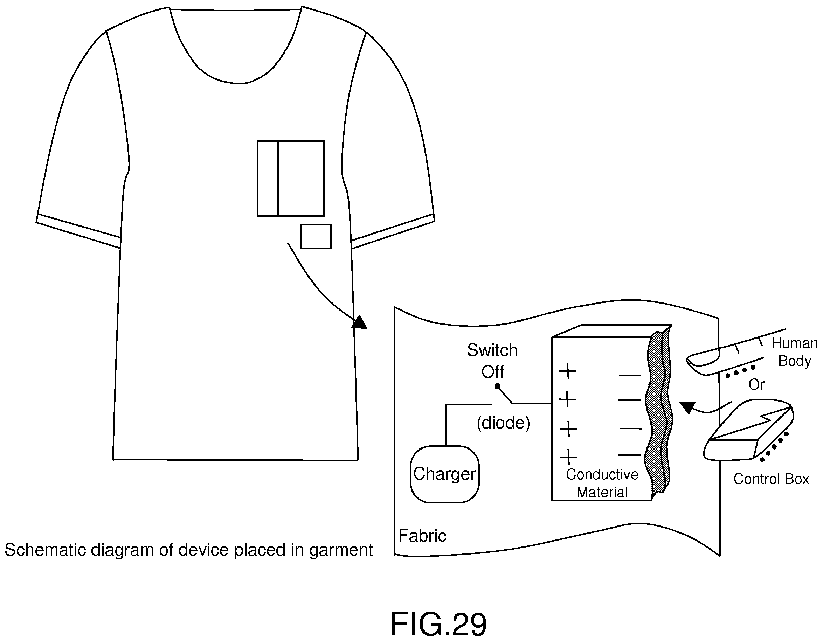

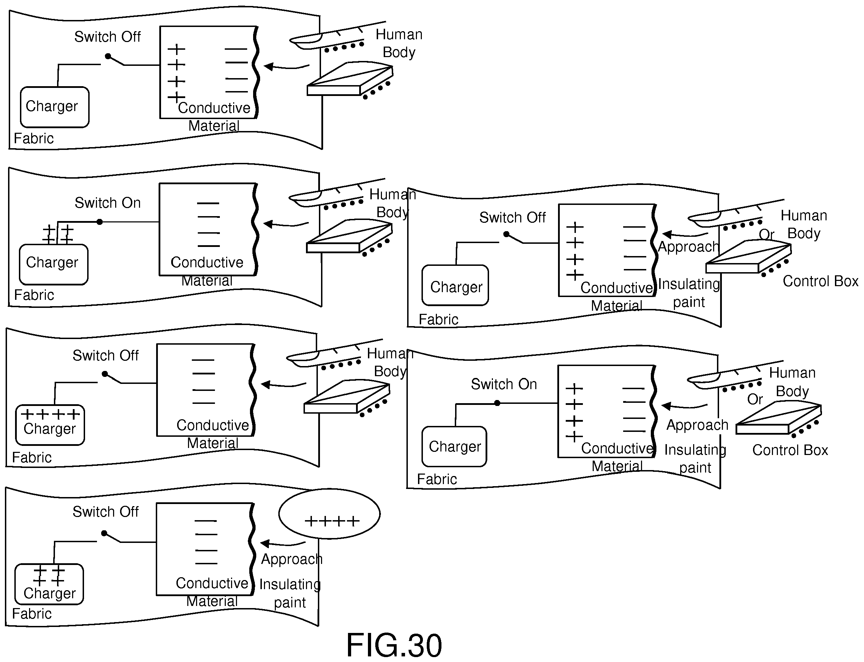

In the device designed by us (FIG. 29, FIG. 30), the person is a live conductor and the control box is also positively charged (similarly, negatively charged). The outer edge of the conductor or fabric is coated with an insulating paint. When the human body or the control box is in contact with the conductive material, the adsorption effect will cause the conductive material or the surrounding fabric to be negatively charged, so that the electricity can be transmitted to the external charger. When the charger's connection switch is turned on, positively charged air enters and continues to charge. When the wireless remote control device is controlled to turn off the power of the charger, Coulomb's law can be used to measure the distance between the person and the conductive material. Under constant charging and measurement, the charger can be used as a storage battery, and will be restored after being turned off.

The switch can also be changed to a diode to selectively charge positive/negative charge. The switches of FIG. 29 and FIG. 30 can also be made of fabric, as the previous patent US8331077 or US20100170704 A1, in which the conductive areas on one side is connected to an electrostatic conductive material, and the conductive area on the other side is connected to the charger connector. As long as there is static electricity to generate, the two conductive areas will react and charge! For example, FIG. 19 of US201001704A1, in which 93a and 92 are the fabric switch and the electrostatic conductive material, and 93b is the charger connector. Therefore, as long as there is static electricity, 92 and 93a will repel and separate, and 92 will be connected with 93b to make static electricity flow into the charger.

Similarly, the charger (capacitor) here can also be used as a sensor, that is to say, the conductive fabric above the charger can sense the change of static electricity, and can also infer the change of humidity. Conductive fabric can also be replaced by wires on the fabric. As long as there is induction, the wire can be connected to the charger (capacitor), and the change in the capacitance value is sensed to know the change in static electricity between the fabric and the human body. Since the capacitance capacity varies with the quantity of static electricity, the change measured in capacitance is the change in the quantity of static electricity. The measurement method is same as in the previous patent US 20130066168 A1.

The charger can be changed into a light-emitting material such as a light-emitting diode, an LED, a tungsten wire, etc., and the electric quantity can be expressed by the brightness, thereby eliminating the static electricity. It can also be changed into a heating wire to provide a heat insulating effect.

Therefore, this concept can also be formed under the contact of two or more pieces of fabric. Two pieces of conductive materials or fabrics are coated with an insulating paint on the outside. When a positively charged human body or control box contacts the sensing conductor, the adsorption effect causes the conductor or surrounding fabric to be negatively charged.

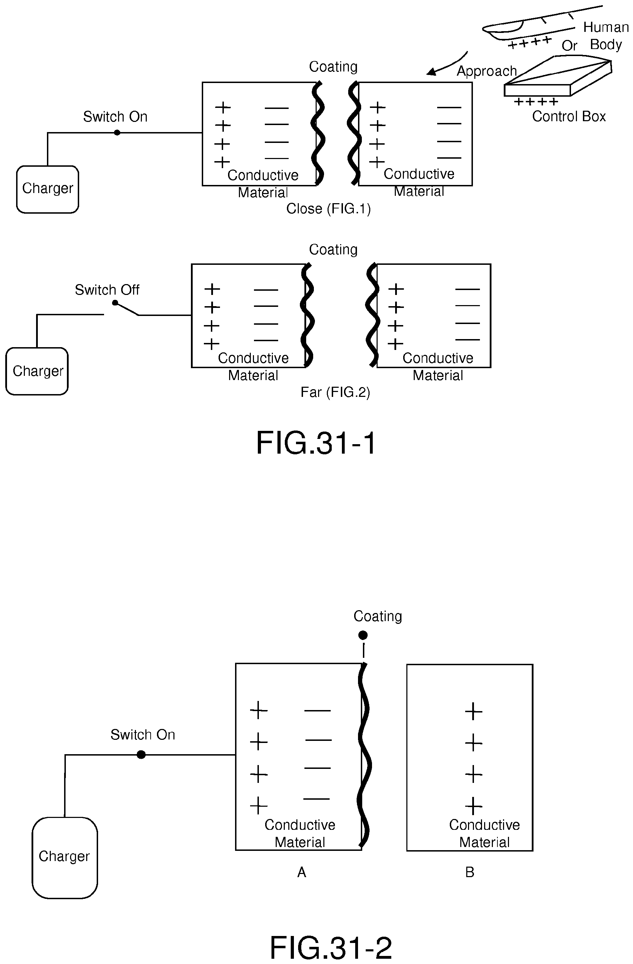

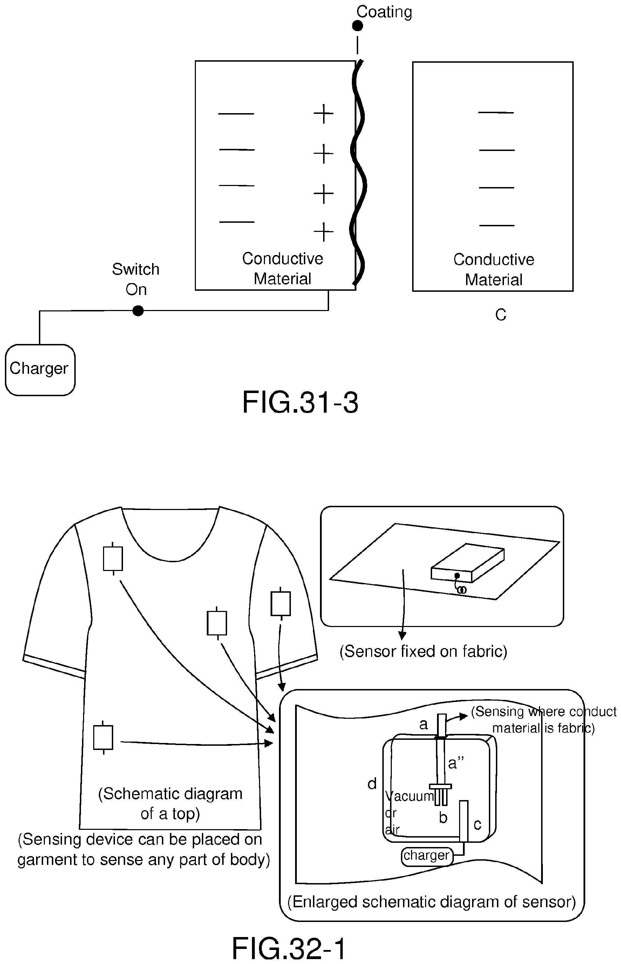

The conductive material on the edge of the charger is positively charged, so the charger can collect the electricity. The two pieces of fabric are continuously inductive and continuously charged, so that they can serve as the storage battery (as shown in FIG. 31-1). When the charger switch is turned off, the conductive materials will be far away from each other at one moment. This sensor can read the measured distance in Coulomb's law (as shown in FIG. 31-1, FIG. 31-2, FIG. 31-3.), which actually reads the change of electric quantity in capacitor or battery.

The human body often produces static electricity due to the friction of the fabric, especially when it is dry and cold. Animal fur also produces static electricity due to friction.

In addition, we are designing an electrostatic sensor, like a traditional electroscope, whose structure is shown in FIG. 32-1. It has a closed space in the fabric and contains two pieces of conductive materials (such as conductive wires or conductive sheets). Its known technology can detect:
Whether the object to be tested is charged, the electrical property of the object to be tested, and whether the object to be tested is a conductor.

It can also detect the movements, breathing, swallowing and other behaviors of the human body.

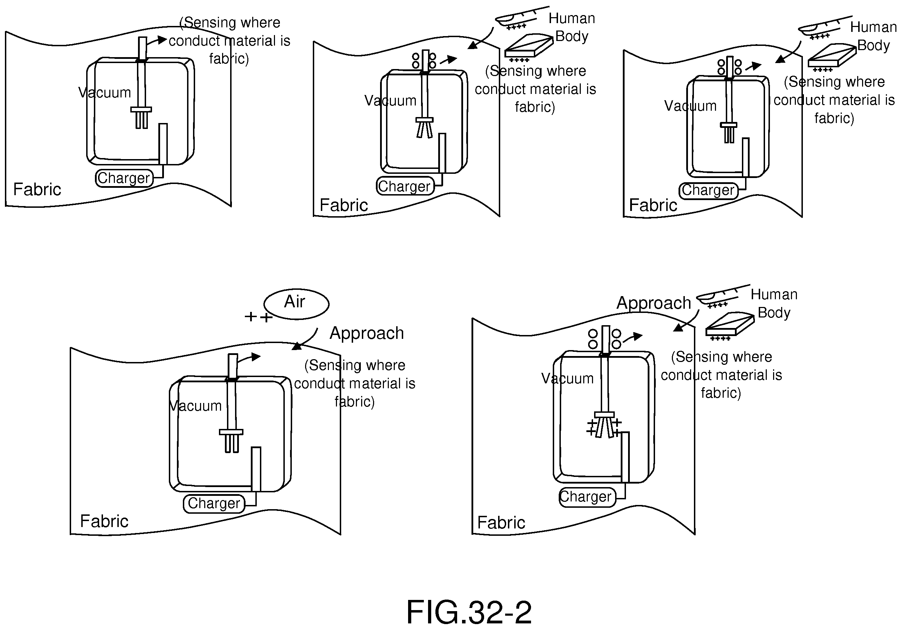

We designed an electrostatic sensor, in which the conductive material a (fabric or wire) is on the fabric, and the conductive column a" communicates with the conductive material a (as shown in FIG. 32-1, FIG. 32-2). The reason for the vacuum is to enhance the sensitivity of shrapnel switch b. It also may not be vacuum, but the efficiency is deteriorated, for example, the air is in the closed space. The outer wall d of the closed space is made of an insulating material, and the conductive material c can be placed in the closed space to discharge the static electricity. When the battery (capacitor) is connected, it can be charged. At the same time, the static interference is eliminated, and the change of the external static electricity can be known from the variation of the capacitor.

When there is no external force, the shrapnel switch (b) is closed and not charged. When the positively charged human body, air or the control box contacts the induction, the shrapnel switch b automatically turns in positive charge, and the shrapnel (b) will touch the conductive material (c) to charge.

An additional remote or passive component can be added. When the battery is fully charged, the conductive material c can be moved down without touching the switch. If the control box does not touch the iron switch, the upper detection will become sensing value of sensor. By the positive charge in the air, it is automatically returned, and so repeatedly, it becomes an automatic switch and automatically charges. The above-described shrapnel switch b is, for example, a tin foil, a metal wire or a conductive wire. The conductive material c is, for example, an iron piece, and the insulating material d can also be changed to a conductive material c, at this time, it can replace the conductive material c, and this sensor can be placed outside the fabric or in the middle of the fabric.

That is to say, the conductive fabric (wire) a, when it is energized, the two pieces of conductive sheets (b) are separately contacted to the conductive material (d) of the outer wall to allow electricity to transmit to the battery (capacitor).

Another electrometer is also a closed space on the cloth as shown in FIG. (32-1), only two of the shrapnels are changed into a rotatable conductive strip, and the outer wall is also made of conductive material. Analyzed from the structural, it is a variable capacitor. Like the structure of the Braun electrostatics, we just do it on the fabric. When it is energized, the rotatable conductive strip can be charged when it touches the outer wall. Since the electrometer itself is also a capacitor, the electrometer is charged when it is connected in parallel at two points where the potential difference is not zero in the DC circuit. At the same time, a charger (capacitor) is connected to charge.

The following is a sort table for the loss/acquisition of electrons in common substances. It can be seen that:
Substance that are easily positively charged (substance that easily lose electrons):
   Such as: air/asbestos/rabbit hair/glass/human hair/mica/nylon/wool/silk/aluminum/ paper/cotton
Substances that are easily negatively charged (substances that are easily acquire electrons):
   Such as: wood/amber/balloon/gold/styrofoam/acrylic/Scotch tape/PVC/silicon/Teflon/

If two different substances generate static electricity due to contact with each other, this is called contact electrification. The triboelectric effect is one of contact electrification effect. Insulators and non-conducting objects are excellent materials for electrification (generation of static electricity) and retention of charge. The conductor will also generate static electricity. Because conductive objects are prone to lose charge, an insulator must be coated in order to retain the charge

### Application and detection of static electricity:

Physical activity inevitably generates static electricity on the body. Although static electricity can damage electronic components, it can also be used to improve the quality of sensing. In addition to those described in the above table, it is also possible to use a suitable material and body friction on different materials to generate static electricity in the vicinity of the electrode, or to generate static electricity by producing a high voltage by an induction or transformer oscillating circuit so as promote the electrode to be in closer contact with the body and reduce body motion interference. Positive static electricity can attract negative ions in the air, that is, becoming an air cleaner.

The principle is that the voltage of 110v is input, and the rectifying and booster circuit passing the negative ion generator generates a high voltage of -2.5KV at the end of the carbon fiber brush of the generator. Because the current is extremely small, it does not cause dangerous damage to the human body. In order to ionize the neutral atoms at high voltage, the air is decomposed into positive and negative ions. The positive ions of electron are heavier and absorbed by the brush to enter the circulating current. The negative ions are lighter and will be sent to the air along with the wind.

Therefore, the addition of negative ions in the air not only neutralizes and fills the positive ions in the air, making the body more energetic and the cells active, but also can also absorb impurities and smoke in the air. As a result, the air that is breathed can be cleaner, and the environment has also become clean.

The present invention is an electrometer with a closed space bottle as shown in FIG. 32-1. It can also be made on the fabric to have a closed space for detecting static electricity. The frequency at which the tin foil (b) in the electrometer is opened and closed represents the intensity of static electricity. This electrometer is also an anion sensor in the air that can detect the parameters of the wearer's environment and human factors. For example, the friction between two pieces of fabric of different materials will generate static electricity. Measuring the static electricity of each part on the body can know the activity of such part.

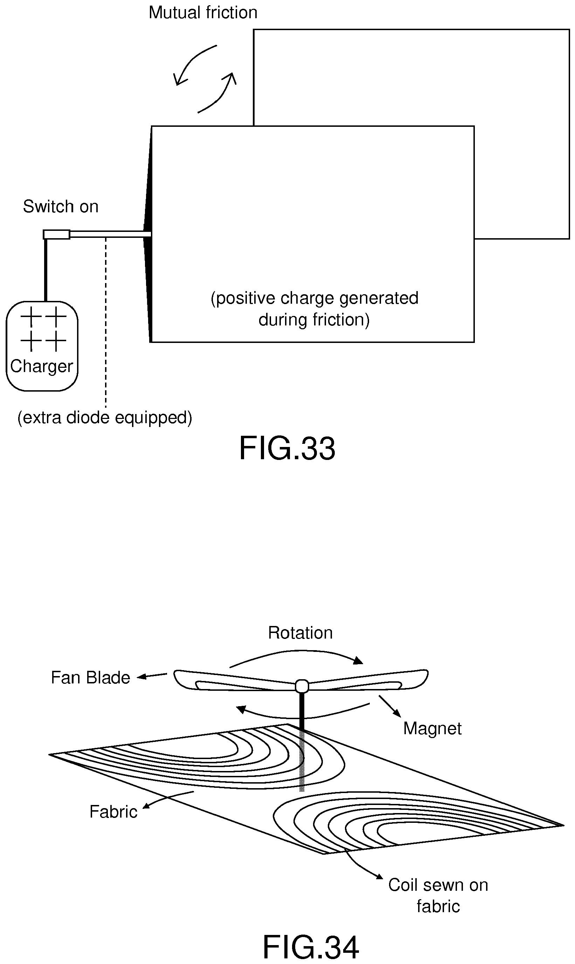

Because friction can generate static electricity (FIG. 33.), we may charge the charger (capacitor) that stores static electricity to remove the static electricity. After the charging switch is turned off, the two materials are in opposite polarity and can sense the distance. Under the interaction of the two pieces of fabric, you can know whether it is dynamic or static. When you move, you will generate electricity and you will know if the user has signs of life. The positive charge in the air is always endless, and it will collect the electricity. It is more environmentally friendly to use air circulation to replenish electricity, so electricity is generated by friction between air and fabric.

A conductive fabric can be arranged at the intersection of fabric, pants, socks and shoes in the present invention. A battery or a large capacitor is arranged on the fabric, and the GND of the static electricity (collecting circuit) is connected to the shoe. A conductor is placed on the sole to contact the ground, and a transistor is arranged between the GND and the sole conductor for intermittent conducting, for example, 0.01 second conducting for one minute, which can collect electrostatic energy.

Magnets and coils can be arranged on the control box, the fabric, the belt, the necklace or the shoes & socks in the present invention. When there is relative motion (that is, the coil cuts the magnetic field lines), it can induce the electricity generation. As shown in FIG. 34, the wind generated by the motion causes the magnetic material or coil to rotate to produce continuous electrical energy. Thermocouples can also generate electricity in the presence of temperature differences. It can generate electricity by using body temperature. The thermocouple can also heat or cool after being energized. Piezoelectric (PZT) can also generate electricity under pressure.

The application of flexible solar cells placed on the outer fabric can also generate electricity for use in the above circuits.

The electric energy generated in the above manner can be connected to the battery (capacitor) on the fabric for storage by the diodes through the electrical conductors on the fabric or connect to the batteries on the control box.

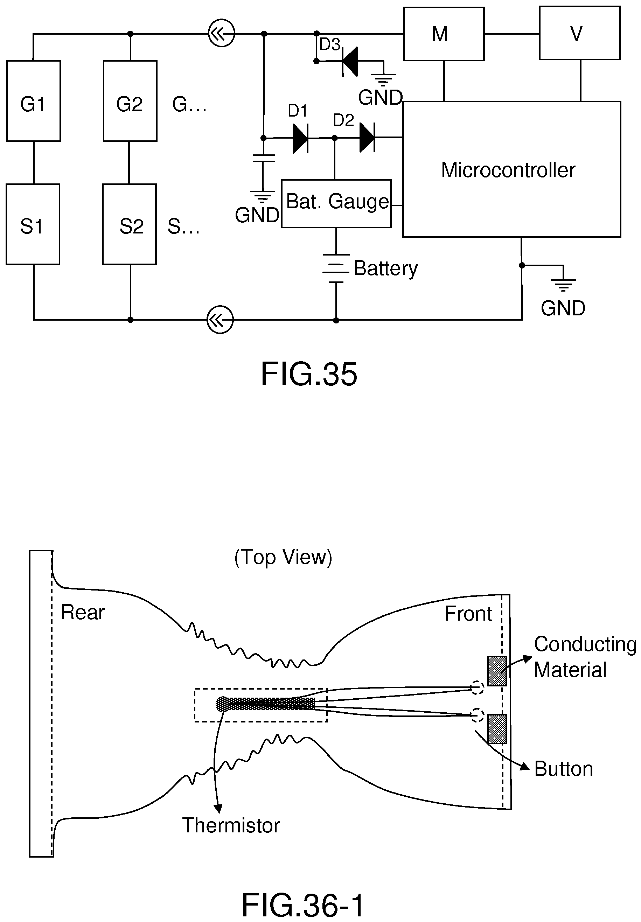

The above-mentioned action of generating electric energy also represents the movement of the human body or the change of the environment. The present invention can selectively add a battery fuel gauge (FIG. 35) integrated circuit to record the amount of charge, that is, record the movement of the human body or the environment change (such as washing, drying, etc.). From the activity of human body and the environmental factors, we can estimate the loss of materials on the wearable sensor and the degree of degradation on the sensing characteristics (such as the elastic fabric of the crack sensor) so as to modify the its response curve, that is, it can improve its accuracy and service life. The battery fuel gauge can also warn you when the power is exhausted (for example, the remaining electric quantity is 5% of the battery capacity).

Tenth Embodiment: an embodiment of a diaper capable of simultaneously measuring urination and defecation, change in breathing and temperature, and posture

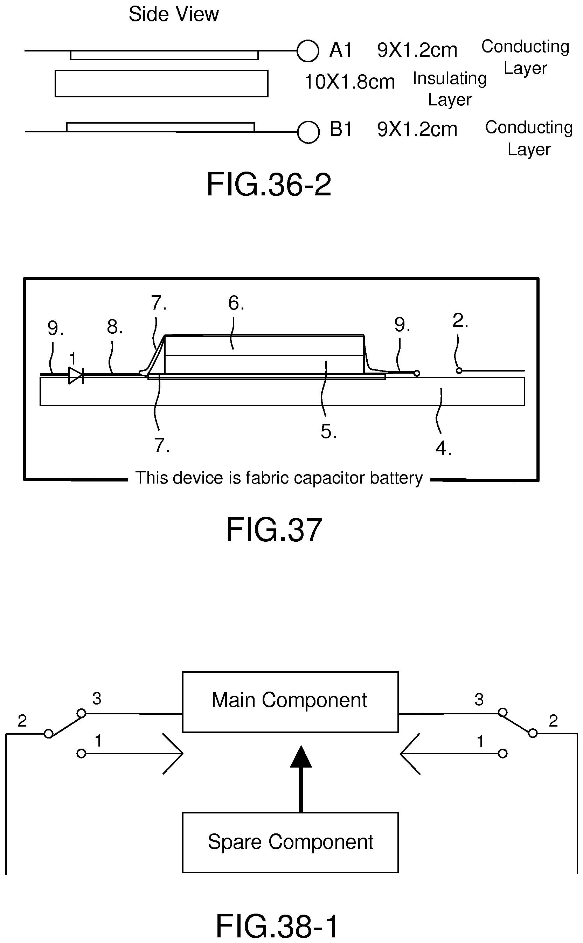

As shown in FIG. 36-1 and FIG. 36-2, A1 is a transmission wire, and the exposed length is 1cm at the end. B1 is also a transmission wire, and the exposed length is 1CM. The distance between A1 and B1 is 10 cm. At the same time, a conductive material (such as conductive silicone) is placed in the middle of the transmission were each. A calcium silicate film is in the middle of the two conductive silicone, that is, three layers are overlaid together. The upper and lower are conductive silicone, which are placed on the bottom layer of diaper. The upper and lower two transmission wires A1 and B1 are connected to the contact points, and a hard frame can be added to prevent the change in reading of the variable capacitors due to being squeezed on both sides. This structure is shown in FIG. (36-2).

**Multifunctional diaper test chart (A1.)**

| Tester | Not touch the breathing electrode | In case of a foreign matter | Breath in sitting | Breath in lying down | Breath in standing | Urine wet test 300ml |
|---|---|---|---|---|---|---|
| A | 220-226nf | 600-680 nf | 516-525 nf | 537-540 nf | 401-410 nf | >2uf |
| B | 240-258 nf | 660-670 nf | 440-445nf | 356-368 nf | 417-423 nf | >2uf |

Referring to the multifunctional fabric chart A1. If the value measured by variable capacitor is basically 200∼500pf when weight of adult is 60 kg, and when there is a foreign matter, about 10 grams will produce 800-1200pf, which can be used to measure the generation of defecation. In addition, when there is no micturition desire, the value of two exposed wires at the front end is close to 30∼80pf. If there is 300cc of micturition desire, the value will reach 2uF, so the smart diaper can multi-functionally detect the user's urination/defecation or sitting down, that is to say, when he stands up and has no micturition desire, the value will be less than 100pf, and if sitting, the value will 200∼500pf. At the same time, it can be washed repeatedly, as shown in the figure. There is conductive material to let the reusable diaper and the button are conducted to connect with the controller, and it can also be made in disposable type. In addition, two sensing electrodes are attached to the waist position to measure changes in the user's breathing. The user's posture change can be detected because the breathing changes are different when the user is standing, sitting or lying down. If the device fails to sense the breathing while the user wears it, it can alert or remind the carer to perform emergency treatment. In addition, the thermistor of this device can also sense changes in the user's body temperature. (As shown in FIG. 36-1 and FIG. 36-2).

If the structure shown above is changed to 1cm*12cm two twilled plain woven silver fabrics spaced at 1.5cm, and is placed in the center of the lower layer of the non-woven fabric on the diaper surface layer. The two pieces of conductive wires are connected to the switch button. In the actual measurement, there is 5g wet defecation without human body pressure, and wetness sensed value is 3nf, and urine wetness displayed value is 2uf.

The above is just an embodiment. We can adjust the sensing materials, the relative position between the sensing materials, the placement position and size of sensing materials, and then the displayed value has different effects.

Similarly, the above example of diaper can adjust the urine, the urination/defecation, the breathing effect and the posture changes (such as: lying down, standing, sitting) in the structure.

The above method can also be used to measure breathing with electrodes, just like a capacitive patent (US 2013/0066168 A1). In addition, the electrode can also be used to measure ECG, heartbeat, sweat, etc. to replace or simultaneously measure breathing.

### Eleventh Embodiment: Capacitor battery (FIG. 37)

Number in the figure: 1. Diode 2. Switch 3. Fabric capacitor battery 4. Fabric
5. Conductive silicone 6. Plain woven silver fabric 7. Packaging film 8. Conductive wire 9. Conductive wire

As shown in FIG. 37, the fabric capacitor battery can store 180uf of electricity. Its area is 1.5*2.5cm² of plain woven silver fabric. The conductive silicone in the area 1.5*2.5cm² is overlaid on it, and there is a packaging film on its upper and lower sides, then the battery is completed. The mechanism of charging and discharging can be achieved by connecting the diode to one end of the wire and connecting the other end to the switch, as shown in FIG. 32.

### Part 2: Wireless signal transmission

The system disclosed by the present invention preferentially uses the wired signal transmission when the wired communication can work normally, and starts the wireless signal transmission when the wired signal transmission fails.

There are often overlapping or contiguous parts on the garments, such as shoes and socks, socks and pants, pants and garment, underwear and shirts, etc., the signals of which can be transmitted by NFC (near field communication), or the signals are transmitted using capacitive coupling, inductive coupling, magnetic coupling, or optical coupling (LEDs and photodiodes).

Adopt a conventional NFC technology, such as RFID, to arrange a digital circuit (microcontroller, memory, such as SD memory, battery, coil, etc., can be fastened to the garment with a snap-fastener, in a way similar to the hung components as described in Part 4), and the signals are transferred from the garment to the control box in a digital mode. The digital circuit contains a memory, so that when the control box is not turned on or too busy to receive the signals, the signals can be digitized first and then stored in the memory, until the control box can receive the signals. The control box can also upload the signals received to a cloud database, to ensure that the data will not be lost even if the control box is damaged.

Transmitting a signal through a capacitor, inductor, magnetic coupling or optical coupling has two advantages: firstly, it is not required to physically connect with a wire or connector (such as a button), and it is convenient, comfortable, and unconstrained for use. Secondly, an analog or digital signal can be transmitted, or can even be applied to the previous embodiment, as long as the coupling is strong enough and the frequency is high enough, namely the digital circuit and connector can be omitted, which is convenient and comfortable for the user.

In the case of inductive coupling and coiled NFC, the present invention adds a magnetic shielding fabric (combined with a magnetic conductive substance, such as ferrite) between the human body and the inductive coil to prevent the magnetic field from affecting the human body function. Optionally, a coil can be added between the magnetic shielding fabric and the human body to sense whether there is a magnetic field or electromagnetic wave before transmission. If there is, it means that the magnetic shielding fabric is invalid, or when strong electromagnetic waves are detected outside, the control box will warn and remind the user to stay away. The magnetic shielding fabric can be attached to the fabric with a removable hook & loop.

### Part 3: Increase reliability with spare components

In the present invention, an important component can be connected in parallel with a spare component socket (as shown in FIG. 38-1), each end of which is a single-pole double-throw switch, wherein the pole (Position 2) is a conductive spring piece. When the spare component is not inserted, this pole will be stuck in Position 3 to connect the main component (Position 3). When the main component fails, the user can insert the spare component and fix the pole at Position 1, to disconnect the main component from the original circuit, and connect the standby component to the original circuit.

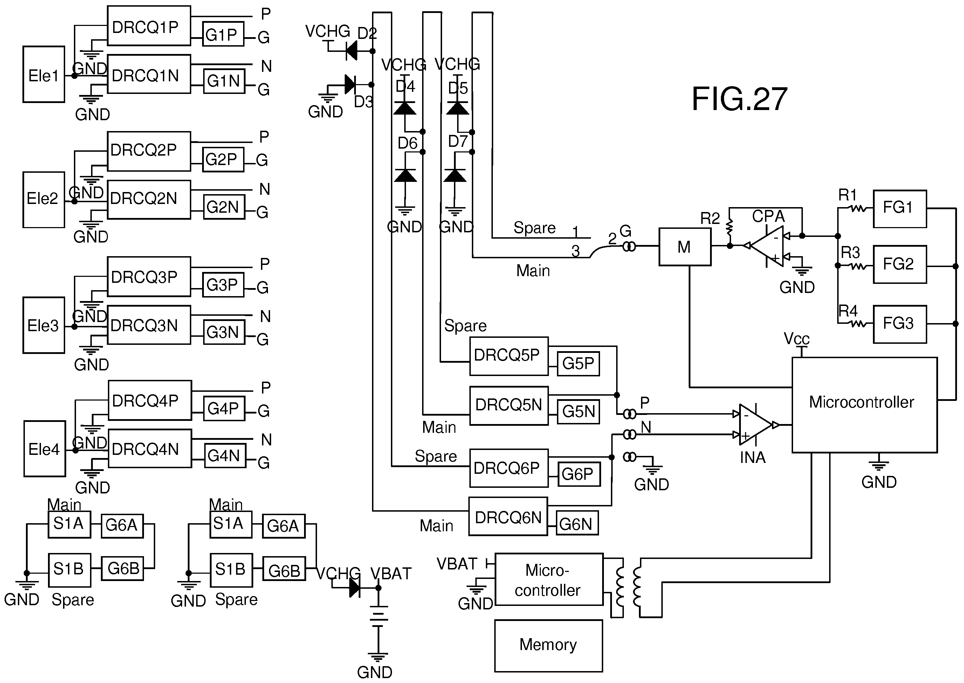

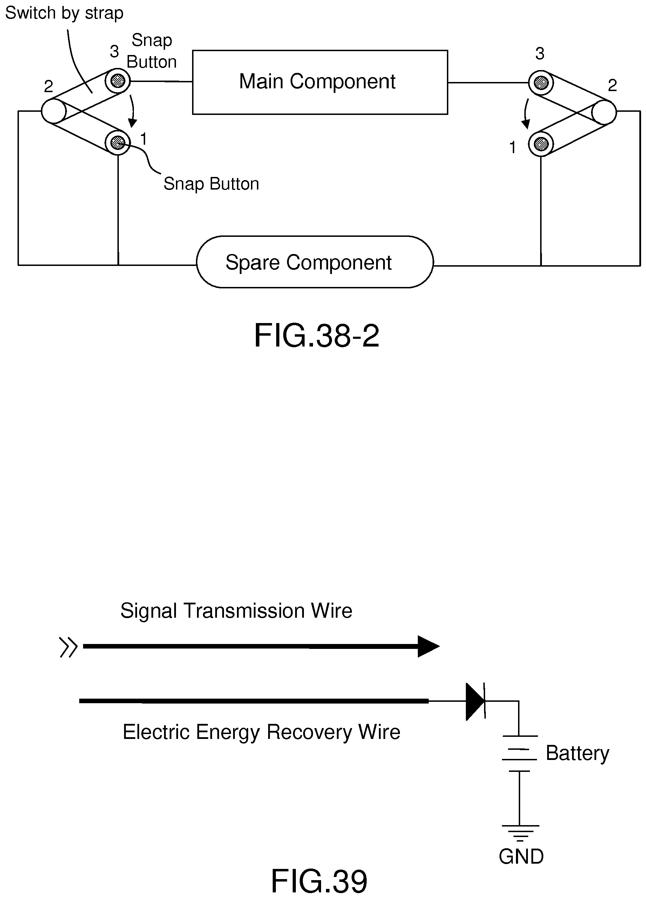

A garment-based wearable sensor must be washed, and the components on the garment (conducting wire, sensing component, integrated circuit, etc.) are easily damaged, so that the present invention is provided with a functionally identical or similar spare component on the garment connected in series or parallel with the main component, as shown in FIG. 38-2. The spare component and the main component can be connected to different selectors, so that the microcontroller can select the spare component or the main component as the case may be. The entire backup system is shown in FIG. 27.

Taking the wire as an example, the spare component is arranged close to the main component, to realize the sensing function. If the main wire is a bare wire (without insulating layer) containing metal (such as silver), the spare wire is an insulated wire (with insulating layer).

### Part 4: Electronization and maintenance of fabric

There are two methods to electronize the fabric. The first method is to use an external component (i.e. a traditional packaged electronic component) with pins bare, and then fix to the fabric by using the textile techniques disclosed in the prior patents (such as stitching). By using this method, the electronic components can be repaired in accordance with conventional electronic and textile techniques, such as cutting the original connecting wires, and then fixing the components and connecting wires with the heat shrinkable sleeves.

As for the second method, the fabric is used as a carrier to integrate the electronic components (such as resistors, transistors, switches, diodes, antennas, etc.) on the fabric by smearing, printing, etching, lithography, etc., similar to the fabrication of flexible liquid crystals. Taking a capacitor as an example, a conductive ink containing graphite can be printed on the fabric, and the dielectric can also be applied to the conductive ink, and then another layer of conductive ink is printed to form a capacitor. Taking a resistor as an example, a conductive ink containing graphite may be printed on the fabric, and a resistive material may also be coated on the conductive ink, and then another layer of conductive ink is printed to form a resistor. In this way, the component is not easy for replacing, and if it is damaged, it must be replaced by an external component. In the garment, a socket can be prearranged, and the socket is provided with a sheath. When the external component is inserted, the original component is no longer connected. The advantage of one-piece integration is that it is more aesthetic (the components are invisible), and the disadvantage is that it is not easy to disassemble.

The sensor on the fabric may age due to factors such as drying, washing, body friction, etc., and it needs to be corrected again, that is, the calibration curve of the database is to be modified. The present invention can predict the coefficient of aging over time, or take the normal activities (e.g., breathing) as default, and then make adjustments according to the extent of each physical activity. Or take the "not worn" (not tensioned) basic values as the baseline, or take fixed external force (such as the washing force of a washing machine) as a standard value for routine calibration.

### Part 5: Multifunctional spare wires

Taking the wire as an example, the spare component is arranged close to the main component, to realize the sensing function. If the main wire is a bare yarn (without insulating layer) containing metal (such as silver), the spare wire is an insulated wire (with insulating layer).

One end of the insulated wire is connected in series with the above mentioned selector. In normal operation, the bare conductive yarn is used first. If the ECG signal is abnormal (for example, the signal is too weak or the noise is too strong), and the temperature and humidity are not low, there may be two reasons: one is that the conductive bare yarn is broken, and the other is that sweating causes short-circuit of ECG signal. At this point, the microcontroller can switch the selector to measure the DC resistance between adjacent conductive bare yarns. If it is low, it is known to be sweaty. If it is high, it is known to be an open circuit. At this time, it can be switched to the spare wire (insulated wire). On the other hand, for the condition caused by sweat, local heating can be caused by a large current to accelerate the evaporation of sweat, and the DC resistance can be re-measured at a certain interval (for example, 60 seconds) until the body surface sweat is evaporated. The time taken to dry the sweat can also be regarded as the degree of sweat. The microcontroller can accumulate the degree of sweat. If the degree of sweat reaches a certain level, it means that the degree of sweat is too high, in which case the user will be reminded of to drink water.

The wire can also be made of a specific alloy whose conductivity changes with the outside temperature and can be used to sense body temperature or ambient temperature. In addition, the thermistor of this device can also sense changes in the user's body temperature. Some conductive plastic materials, such as LDPE conductive carbon black, are known to have electrical resistance that varies with temperature. Therefore, it can be used as a conductive transmission wire and a temperature sensor or as a heater. As the temperature drops, its resistance decreases.

The spare wire itself can be used as a sweat or temperature sensor in addition to replacing the broken main conductor. The spare wire can be installed on different parts of the garment, and each spare wire can be switched by the system by an electronic selector (see Part 1), or a turntable or a drawstring can be set on the garment for the user to manually switch. To sense a part, the system automatically or the user manually switches to the spare wire where the part is located. Several spare wires may be arranged in parallel or in a grid shape, vertically in the inner layer and horizontally in the outer layer. When a vertical spare wire and a horizontal spare wire are selected, the degree of sweat can be detected for a specified position.

Regarding the above-mentioned drawstring mechanism, the string can be pulled manually or by a cylinder, hydraulic cylinder or threaded rod.

Spare wires in different layers of spare wires can be bare wires, used to selectively transmit physiological signals or environmental signals of a certain part, such as sweat.

For the human body signal (such as electrophysiological signal), the above-mentioned grid-shaped vertical and horizontal spare wires can also be used to select a specific part. The signal is transmitted from the underwear to the outerwear, transmitted to the chair or bed where the user sits or lies in, then analyzed and processed by the control box installed on the chair or bed. For example, as shown in FIG. 21, the original optical fiber can be replaced by a common transmission wire to achieve the same effects. There are three outerwear in total. The bare transmission wire on the right side of the innermost layer is a vertical transmission wire, such as silver yarn. The bare transmission wire on the right side of the second layer is arranged horizontally, so that the innermost layer and the second layer are electrically connected with each other, and the signal can be transmitted from the inner layer to the second layer. The bare transmission wire of the innermost layer is vertical, and the bare transmission wire of the second layer is also vertical. The two transmission wires are kept at a considerable distance and will not contact with each other. Therefore, the transmission wires only transmit their own signals respectively and will not be interfered. Similarly, in the second layer and the outermost layer, the transmission wires are perpendicular to each other, so that the signal of the second layer will be transmitted to the outermost layer. Therefore, the innermost signal can be transmitted to the outermost layer, and the signals transmitted through the layers respectively will not interfere with each other. At the same time, the transmission wire can also be made into different forms, not necessarily vertical, such as a concentric circle or a radial shape. The purpose is to use such wire as an antenna to transmit the signal, just in the front of garment, so that the inner layer signal can also be transmitted to the outermost layer upon the principle of the antenna. Therefore, as long as the bare wire on our garment does not break, we can also have an insulated wire used as a backup when the bare wire fails. At the same time, the signal generated on the bare wire allows us to know that the relevant connectivity between the layers of fabric, namely when we do more physical exercises, the noise generated from the contact between the two layers will increase. If we sweat, the effect of the relative signal conduction will be getting better, but the separate individual outgoing signal wire on the back will be getting worse. Thus, the human body changes with the outside world can be learnt about, that is, the transmission will be better when the external environment temperature is too high or we sweat. In winter, the skin is dry and the body temperature is low, so that the transmission of both wires will get worse. On the contrary, the effect of the transmission signals on the back can be interfered easily.

As for the wearable applications, electricity is an important factor. In order to ensure sufficient power supply, the present invention can connect an electric energy recovery wire (FIG. 39) next to the signal transmission wire, which is connected with the diode and battery (capacitor). When the signal passes, the mutual inductance or coupling capacitance between the two wires is used to induce the electric energy on the electric energy recovery wire, and then such energy will be stored by the battery or the capacitor for later use.

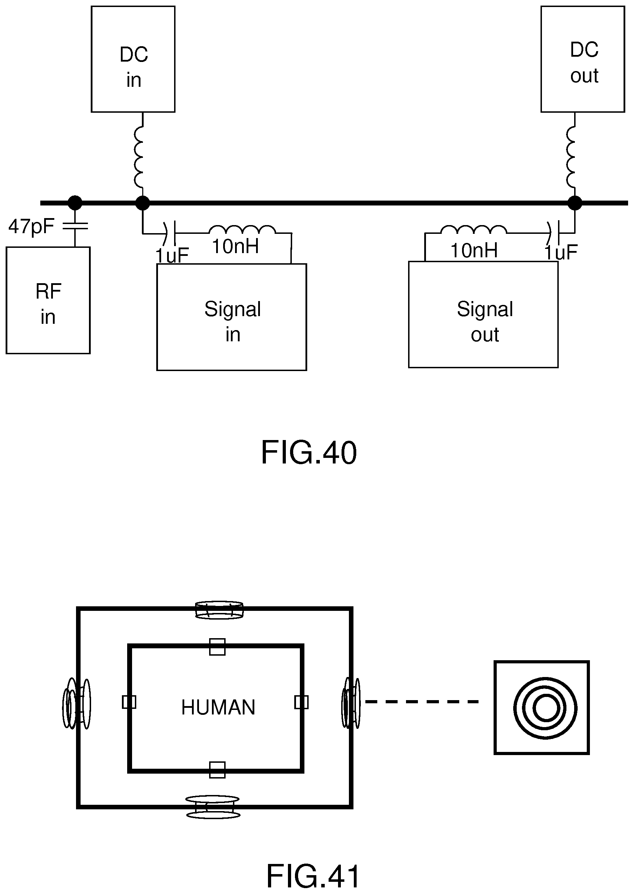

The transmission wire can also serve as a radio frequency (RF) antenna, power transmission wire (DC), and transmission wire for the sensing signals (AC) (as shown in FIG. 40). Usually the RF frequency is higher than 200MHz, so that a 47pF can be used for feeding RF into the transmission wire, while isolating the DC and sensing signals. Usually, the frequency of the sensing signal is about 1Hz∼1MHz. Accordingly, a 1uF can be used for the sensing signal can be used for feeding the sensing signal into the transmission wire, as well as isolating the DC power supply, and connecting in series with a small inducer to isolate the radio frequency (RF). As shown in the above figure, the signal can be transmitted to the outerwear from the underwear. As shown in FIG. 41, the transmission wire can also serve as an antenna. Similarly, it can also be used to transmit the signals from the chest sensor to the control box arrange at the abdomen or on a chair or bed, or transmit the signal from the upper arm sensor to the control box of the lower arm. The strength of the received signal can be used to measure the distance between the sending and receiving positions, to determine the relative movement, that is, it can be used for detecting the breathing, body motion, posture or heartbeat. At the same time, it is also possible to read the intensity of external electromagnetic waves, such as the electromagnetic waves generated by a nearby base station or mobile phone.

In the above example, the transmission wire is also used as a radio frequency (RF) antenna, which will inevitably interfere with the physiological signals or the signals generated from various sensors on the body. For example, the commonly used Bluetooth communication adopts the SFK (Shift Frequency Key) technology to convert between 0 and 1, and the instantaneous interference is not small. In order to reduce such interference, the amplitude modulation (AM) technology is used alternatively, and AM is also better in saving electric energy.

Similarly, it can also be used to transmit the signal obtained from the inductive pneumograph sensor at the chest to the inductive pneumograph sensor at the abdomen or the control box on a chair or bed, or transmit the signal obtained from the inductive pneumograph sensor at the upper arm to the inductive pneumograph sensor at the lower arm.

The transmission wire itself can also be used as an inductive pneumograph sensor. The preferred embodiment is that the wire is arranged in a "Z" shape to make sufficient allowance for expansion and contraction. It can not only be used to inject current, but also be used for transmitting the signal from the outerwear to the underwear by using the wire structure as shown in FIG. 21. As shown in FIG. 21, the different layers of garments have different bare wires that can be in contact with each other. As long as the inductive pneumograph sensor is not covered by the insulation layer, this method can be used to inject current or read the inductance value. In addition to direct contact, the inductive pneumograph sensor and transmission wire can also transmit signals by inductive coupling, capacitive coupling or electromagnetic wave transmission. It is advantageous to place the control box in the outerwear: it is unlikely to be influenced by the body motion. The inductive pneumograph sensor can be wound for more times. The self-inductance of each sensor represents the expansion or contraction at this position. The mutual inductance can represent the relative position change of each part, and the position change represents relative movement. Similarly, the coupling capacitance between the capacitive sensor and the wire can also be used to represent the relative position change of each part (FIG. 9-1 & 9-2):
The above-mentioned grid lines can also be used to measure changes in the local part of body, for example, injecting a current into the entire coil and observing the self-inductance value of the entire coil to determine the changes in the body circumstance. If only the inductive change of front chest is observed by using the grid line, the result represents only the changes in the local part of chest. Similarly, other parts can be determined by using the same method, or measured separately as shown in the Figure. In addition, as shown in FIG. 9-1 and 9-2, inductive pneumograph sensors can be installed for both the chest and the abdomen respectively, and the body circumference change caused by chest and abdominal breathing can be measured separately, or can also be measured based on the mutual inductance change between the two sensors. The mutual inductance change represents the relative position change between the two sensors. The signals can also be transmitted by using the mutual inductance. For example, when we measure the self-inductance of the chest, the abdominal sensor can also receive the breathing signal due to the mutual inductance, so that the control box can also obtain the chest breathing signal from the abdominal sensor. Similarly, inductive pneumograph sensors can also be arranged on the upper arm and the lower arm respectively, and the change in mutual inductance represents the flexion of the arm. Similarly, inductive pneumograph sensors can be arranged at the chest and arm respectively, and the change in mutual inductance represents the relative movement of the arm and the chest. Similarly, if inductive pneumograph sensors are used for different persons, the mutual inductance between then represents the relative movement between them.

Inductive pneumograph sensors can also be installed on the underwear and outerwear respectively at the same position of body. The mutual inductance between the two sensors represents the relative movement between the underwear and outerwear, such as the action of undressing or dressing, or a backpack pressing there, or the garment being too loose there, or the relative position change being caused by the movement of limb.

Similarly, inductive pneumograph sensors can be placed on a garment and a bed respectively. The mutual inductance between the two sensors can represent the relative movement between this bed and the person wearing this garment, and can be used for sleep monitoring or for representing the relative distance between two persons. Similarly, they can also be used for representing the relative movement between an animal and a person, or between animals.

In the above example, the mutual inductance between the two sensors can also be used for transmitting signals. For example, the chest breathing signal can be transmitted from the underwear to the outerwear, while the control box is connected between the inductive pneumograph sensors installed on the underwear and the outerwear, namely the outerwear can receive the breathing signal from the underwear. Similarly, the underwear can also receive signals from the outerwear via mutual inductance.

In the same way, the above-mentioned mutual inductance based transmission signal technology can also be used to transmit signals by using a coupling capacitor between the wires.

US 7750790 B2 discloses a fabric strain gauge whose impedance changes with stress. The present invention can make a wire by using this strain gauge, and the impedance of this strain gauge also changes with the degree of moisture (sweat or humidity), so that it can also serve as a moisture sensor at the same time. The present invention can utilize the frequency domain to distinguish between breathing and moisture. The respiratory rate is usually greater than 0.1 Hz (six times per minute), while the moisture usually changes very slowly (evaporation usually takes hours), much less than 0.1 Hz. The bandpass filter can be used to distinguish between them. Similarly, the strain gauge can sense movements in other parts of the body, such as raising hands and lifting legs. As in the above-mentioned example, the wire contained in this strain gauge can be made of an alloy with a large temperature coefficient, that is, it can also serve as a thermometer. The impedance of this strain gauge also changes with the electrolyte contained in the moisture (sodium, potassium, chloride ions, laundry detergent), namely it can also be used as an electrolyte sensor. Upon this principle, this strain gauge can also detect changes in the surface of skin, such as wound healing or enlargement, bleeding, iodine application, or residual detergent on garment.

Magnets are arranged in the inner layer of garment, and a coil is placed in the outer layer of garment. The interaction between the magnet and coil will produce an induced current. Therefore, the mutual movement between the two pieces of fabric can be known, and the message can also be transmitted. In principle, the greater the mutual movement between the two magnets and the coil, the greater the current generated. As shown in FIG. 41, if the magnet in the underwear is added with a coil, to form mutual inductance with the coil of the outerwear, for detecting another signal. At the same time, if the innermost layer is provided with a coil other than a magnet, that is, the first layer is provided with a coil, the second layer is provided with a magnet, and the third layer is also provided with a coil, then the reaction between the first layer and the second layer can also be detected. The reaction between the second layer and the third layer can also be detected, and the mutual inductance between the first layer and the third layer can also be detected. By analogy, the placement does not have to be in the same position, that is, the magnet and coils of the first layer and the second layer are not necessarily in the same position as the magnets of the second layer and the third layer. The meaning of measurement is different, that is to say the measured signals represent the inductances and effects of different positions.

Optical fiber has such advantages: it is not susceptible to interference from wireless batteries or electric fields or magnetic fields! Similarly, the two optical fibers are respectively installed in the outer layer and inner layer of the garment, and can also conduct light when touched. That is to say, the above-mentioned effects can also be achieved by using optical fibers.

### Part 6: Method for using fabric to generate electrical energy

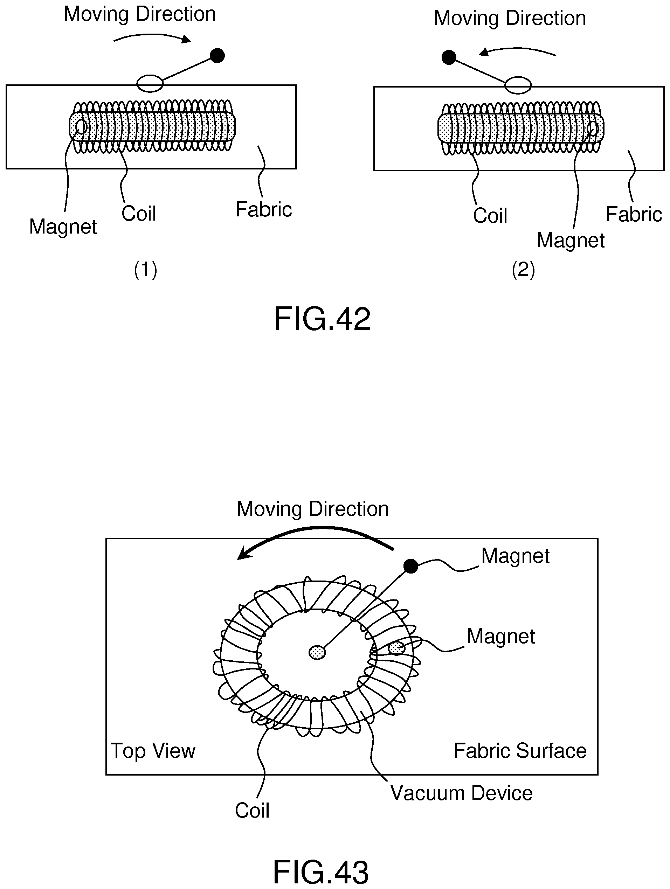

Magnetic materials (conductive materials, made of electromagnetic materials) are oscillated on the fabric to generate electricity. On a piece of fabric, install a sealing device that contains a piece of magnetic material. The sealing device has a winding conductive coil outside, and another magnetic material is mounted on the fabric above the sealing device. The magnetic material can swing freely. Therefore, if the two magnetic materials are same in magnetic poles, and the magnetic material is on the right side, the magnetic material in the sealing device will slide to the left side due to repulsion (as shown in FIG. 42). Similarly, the magnetic material on the left side, the magnetic material will slide to the right in the vacuum (as shown in FIG. 42). The constant changes of the magnetic material on the upper and lower sides will cause the wound coil to generate induced electromotive potential to generate electricity. The sealing device can be either vacuum or inflated. In addition, it is also possible to omit another piece of magnetic material outside the fabric, and only on the fabric, because of the magnetic material in the fabric, it will swing back and forth due to the external force, and electricity can also be generated.

The principle in the following figure (FIG. 43) is the same as described above. The advantage is that the magnetic material placed inside the circular vacuum will always rotate when it is shaken by the external magnetic material, and it cannot be stopped, resulting in continuous power, with better effects. In the same way, it is not necessary to place the magnetic material outside the fabric, or the user can add more than one magnetic material outside the fabric to improve the efficiency.

### Part 7: Normal operating procedures and functions

The normal operating procedure of the present invention begins with the purchase of garment by the user, as described below.
1. The garment itself has a sensor. Once it is moved (taken down from a hanger), the digital circuit on the garment is activated and begins to conduct self-check. In addition, even if it is not subjected to external force, it can detect the entire system from time to time. Thus the user can learn about how long it has been from the time of manufacture to sale, and whether the functions inside have declined. For example, if the foregoing circuit inspection wires and sensors are damaged, the spare components will be used as substitutes, with results displayed. For example, the green light is normal, and the red light means some abnormalities. The control box is turned on, the self-check is performed first, and then the communication with the garment is established. The wired communication is preferentially used. If the wired communication cannot be used, the wireless communication is then used.
2. When a user buys a garment and tries it on, the sensor on the garment will start to work. If it is too loose or too tight, the user will be reminded and recommended with an appropriate size. If there is still a slight discomfort, the user will be reminded to modify and adjust the strap string or the hook & loop, etc., to ensure the optimized effects based on the signals detected.
   It is not necessary to wear the garment for another method. The user can use a 3D dynamic fitting system to dynamically try it on in front of the computer supported dressing mirror to correct the size of the model, and then try it on again before purchase, saving time and improving efficiency.
3. Once the garment is confirmed as fitted, the system will conduct self-correction (passive correction). The results are stored in the memory as described above, and are uploaded to the cloud database at proper time.
4. After the self-correction is completed, the aforementioned energy pick-up mechanism is activated, to determine the physiological parameters (electrocardiogram, respiration, body temperature, etc.) and environmental parameters (room temperature, humidity, etc.) as described above. The physiological and environmental parameters acquired will be uploaded to the cloud database at proper time, and then can be used for long-term trend analysis.
5. The control box checks the signal quality and advises the user to make appropriate adjustments. The controller itself can be placed directly on the garment and be formed in one piece with the garment. Taking the ECG as an example, if the signal/noise ratio is too low, the reason is that the electrode is not properly attached to the human body, and then the user may be advised to slightly tighten the strap to improve the quality of the electrocardiogram. At the same time, based on the database of the aging of the relevant components, the aging of the component can be estimated, and the response curve can be adjusted to obtain the best measurement accuracy. The set values after adjusted will be uploaded to the cloud database at proper time, and then can be used for long-term trend analysis.
6. The information stored in the memory on the digital circuit of garment will be uploaded at proper time as described above, or the memory may be a SD memory or alike which can be connected or disconnected freely.
7. If the user wears it for a long time, or if the ambient temperature or humidity change in a wide range, leading to any potential deviations from the set values, the garment can conduct self-correction automatically.
8. When the user takes off the garment and prepares for washing, the digital circuit in the garment will operate continuously to record the environment conditions, such as washing, drying or being stored in the closet, or the washing power, drying temperature and humidity, etc. These factors will affect the response curve and service life of the sensor. The response curves can be adjusted according to such records, and can be used for estimating the residual life of sensor, to remind the user to repair it at proper time.

## Claims

1. A multifunctional fabric sensing system, comprising: a fabric layer; and a plurality of sensors disposed on the fabric layer, wherein the plurality of sensors comprise up to two output ends, wherein a signal of each of the sensors can be monitored, or electrostatic interference can be removed from the fabric, or detection can be performed without a force being applied.

2. The multifunctional fabric sensing system of claim 1, wherein at least one of the sensors is connected to a selector for detection.

3. The multifunctional fabric sensing system of claim 2, wherein the selector can be a band pass filter, a Zener diode, a rectifier diode, a constant voltage component, a reed switch, a multiplexer and so on.

4. The multifunctional fabric sensing system of claim 1, wherein a charge-consuming component, such as a light-emitting diode, an LED, a tungsten wire or a thermocouple, can be used to remove electrostatic consumption, or a capacitor (battery) can be used to store electrostatic charge for later use.

5. The multifunctional fabric sensing system of claim 1, wherein a pattern or a body painting of a conductive material applied on a skin can be used to remove static electricity.

6. The multifunctional fabric sensing system of claim 1, wherein the sensor is a coil formed by wires wound around an enclosed space in the fabric containing a magnetic material, such as a magnet, therein such that an external force can be sensed while an effect of electromagnetic induction can be realized.

7. The multifunctional fabric sensing system of claim 6, wherein a magnetic material can be equipped outside the fabric or the enclosed space can be pumped into vacuum, and both can enhance the sensing effect.

8. The multifunctional fabric sensing system of claim 1, wherein the sensor can be two conductive sheets disposed in an enclosed space in the fabric, wherein a conductive fabric or a conductive wire outside the fabric is connected to the two conductive sheets so that static electricity can be detected.

9. The multifunctional fabric sensing system of claim 8, wherein an enclosure of the enclosed space can also be a conductive material and can be connected to a capacitor so that it can be used for charging.

10. The multifunctional fabric sensing system of claim 1, wherein the sensor is an electrode that can be realized by a pattern or a painting of a conductive material applied on a skin.

11. The multifunctional fabric sensing system of claim 1, wherein the sensor uses a fabric resistance sensor or an impedance plethysmogram sensor to generate a physiological signal.

12. The multifunctional fabric sensing system of claim 11, wherein the sensor is **characterized in that** it comprises at least one fabric, at least one conductive area is disposed on said fabric, and a signal circuit is provided, wherein the conductive fabric forms a resistance or impedance with a human body, wherein the fabric resistance sensor or the impedance plethysmogram sensor is connected in series or in parallel to a resistor R, a capacitor C, an inductor L, an operational amplifier, a diode, a Schmitt trigger, a complementary metal oxide semiconductor field effect crystal, a transistor, or an integrated circuit to form a charge/discharge circuit to change a frequency, a period, a voltage or a current. When there is pressure, pulling force, torque or tension between the human body and the fabric, said circuit sends a signal and the system receives a change in a value of the resistance sensor or the impedance plethysmogram sensor between the conductive fabric and the human body, and said change is represented by a change of frequency, voltage or current, and at least one of the information of physiology, medium (sweat, blood, lip balm), posture changes, medium changes between the human body and the fabric, or the force received is analyzed through the change in frequency, voltage or current.

13. The multifunctional fabric sensing system of claim 1, wherein the sensor is an electrode, and to measure whether an impedance between the electrode and a body surface is good, an impedance between a single electrode and the body surface is measured.

14. The multifunctional fabric sensing system of claim 13, wherein a stray capacitance is used as a ground wire to measure the impedance.

15. The multifunctional fabric sensing system of claim 1, wherein the sensor is an electrode, and wherein an electrocardiogram and an electromyogram can be obtained simultaneously by using band pass filters with different frequencies.

16. The multifunctional fabric sensing system of claim 1, wherein the sensing electrodes connected in series to an inductor or a capacitor can divide signals by frequency.

17. The multifunctional fabric sensing system of claim 16, wherein for example, an inductor rejects high frequencies but allows low frequencies to pass, a capacitor rejects low frequencies but allows high frequencies to pass, and all frequencies can pass without the inductor and the capacitor, thus three frequency bands can be divided.

18. The multifunctional fabric sensing system of claim 1, wherein the sensor is an inductive pneumograph that can measure breathing or body motion and can also be a transmission wire or antenna.

19. The multifunctional fabric sensing system of claim 14, wherein body fat, sweat, and body water can also be measured, and whether the electrodes are in a good condition is verified.

20. The multifunctional fabric sensing system of claim 1, wherein the sensor is an optical fiber that can transmit signals.

21. The multifunctional fabric sensing system of claim 1, wherein an electric energy recovery wire is disposed close to the transmission wire, and when a signal passes, a mutual induction or a coupling capacitance between the two wires is used to induce electric energy on the electric energy recovery wire.

22. The multifunctional fabric sensing system of claim 1, wherein a battery plus a battery fuel gauge can record an action of charging electric energy, which also represents movement of the human body or environment change.

23. The multifunctional fabric sensing system of claim 1, wherein the system is a diaper sensing system, wherein two bare transmission wires connected to a variable capacitor can measure defecation and urination.

24. The multifunctional fabric sensing system of claim 23, wherein two additional sensing electrodes are attached to a waist position to measure a change in the user's breathing.

25. The multifunctional fabric sensing system of claim 1, wherein the system has more than two layers of fabric, and transmission wires of an inner layer and transmission wires of an outer layer overlap such that signals are transmitted therebetween.

26. The multifunctional fabric sensing system of claim 25, wherein the transmission wires can be bare wires or optical fibers and can also be used antennas for wireless transmission.

27. The multifunctional fabric sensing system of claim 1, wherein the sensor is a capacitor and a conductive fabric or conductive wire connected thereto to detect a change of static electricity, and a change of humidity can also be inferred.

28. The multifunctional fabric sensing system of claim 1, wherein the sensor is an electrode that can measure a physiological signal (ECG, EEG, EMG...) and can also be used to measure sweat by DC or to measure breathing, body motion, posture and body fat by AC.

29. The multifunctional fabric sensing system of claim 1, wherein transmission wires and spare wires can also transmit AC or DC. As a temperature or humidity detector.

30. The multifunctional fabric sensing system of claim 29, wherein transmission wires and spare wires can also use self-induction and mutual induction to read breathing, body motion, posture, heartbeat, etc.

31. A multifunctional fabric sensing method, comprising: a fabric layer; and a plurality of sensors disposed on the fabric layer, wherein the plurality of sensors comprise up to two output ends, wherein a signal of each of the sensors can be monitored, or electrostatic interference can be removed from the fabric, or detection can be performed without a force being applied.

32. A multifunctional fabric sensing article, comprising: a fabric layer; and a plurality of sensors disposed on the fabric layer, wherein the plurality of sensors comprise up to two output ends, wherein a signal of each of the sensors can be monitored, or electrostatic interference can be removed from the fabric, or detection can be performed without a force being applied.
